# EUROPÄISCHE PATENTSCHRIFT

(11) **EP 3 416 612 B1**
(45) Veröffentlichungstag und Bekanntmachung des Hinweises auf die Patenterteilung: **28.12.2022**
(21) Anmeldenummer: 16707654.6
(22) Anmeldetag: 15.02.2016
(51) Int. Cl.: A61K 8/35, A61K 8/37, A61Q 13/00, C11B 9/00, C11D 3/50, A61Q 5/02, A61Q 19/10

(54) **RIECHSTOFFMISCHUNGEN ENTHALTEND ESTER UND KETONE**
FRAGRANT MIXTURES CONTAINING ESTERS AND KETONES
MÉLANGES DE SUBSTANCES ODORANTES CONTENANT DES ESTERS ET DES CÉTONES

(43) Veröffentlichungstag der Anmeldung: 26.12.2018
(73) Patentinhaber: Symrise AG, 37603 Holzminden (DE)
(72) Erfinder: HÖLSCHER, Bernd, 37620 Halle (DE); MANSFELD, Marc, 37647 Brevörde (DE); WAGNER, Tobias, 37627 Hellental (DE); AMOS, Julia, 37632 Eschershausen (DE)
(74) Vertreter: Fabry, Bernd
(86) Internationale Anmeldenummer: PCT/EP2016/053133
(87) Internationale Veröffentlichungsnummer: WO 2017/140336

(56) Entgegenhaltungen:
- EP-A1- 2 268 779
- EP-A1- 2 474 301
- EP-A2- 0 115 278
- DE-A1- 2 729 121
- DE-C- 580 450
- FR-A- 728 998
- GB-A- 391 579
- SU-A1- 1 694 571
- US-A- 4 280 934
- Orville L Chapman ET AL: "Stereochemical evidence of dual chemoreceptors for an achiral sex pheromone in Lepidoptera", Journal of the American Chemical Society, 1. Juli 1978 (1978-07-01), Seiten 4878-4884, XP055310996, DOI: 10.1021/ja00483a039 Gefunden im Internet: URL:http://pubs.acs.org/doi/pdf/10.1021/ja 00483a039
- Herbert C Brown ET AL: "Reaction of Organoboranes with Ethyl Bromoacetate under the Influence of Potassium t-Butoxide. A Convenient Procedure for the Conversion of Olefins into Esters via Hydroboration", J . Am. Chem. SOC, 1. Januar 1967 (1967-01-01), Seiten 818-820, XP055311038, Gefunden im Internet: URL:http://pubs.acs.org/doi/pdf/10.1021/ja 01005a064 [gefunden am 2016-10-14]
- HUAQING LIU ET AL: "Sequential Reactions of Trimethylsilyldiazomethane with 4-Alkenyl Ketones and Aldehydes Catalyzed by Lewis Bases", ORGANIC LETTERS , 14(23), 6012-6015 CODEN: ORLEF7; ISSN: 1523-7052, Bd. 15, Nr. 12, 21. Juni 2013 (2013-06-21) , Seiten 2974-2977, XP055313220, ISSN: 1523-7060, DOI: 10.1021/ol401124t
- ROGER L. SNOWDEN: "Fragmentation of Homoallylic Alkoxides. Preparation of 1-(3?-cyclopentenyl)-2-alkanones from 2-substituted bicyclo[2.2.1]hept-5-en-2-ols", HELVETICA CHIMICA ACTA, Bd. 66, Nr. 4, 15. Juni 1983 (1983-06-15), Seiten 1031-1038, XP055313247, CH ISSN: 0018-019X, DOI: 10.1002/hlca.19830660405
- TOBIAS KAPFERER ET AL: "Asymmetric Dihydroxylation of [beta],[gamma]-Unsaturated Carboxylic Esterswith Trisubstituted C=C Bonds - Enantioselective Syntheses of Trisubstituted [gamma]-Butyrolactones", EUROPEAN JOURNAL OF ORGANIC CHEMISTRY, Bd. 2006, Nr. 9, 1. Mai 2006 (2006-05-01), Seiten 2119-2133, XP055311030, DE ISSN: 1434-193X, DOI: 10.1002/ejoc.200500963
- TABER D. F. ET AL.: "A simple preparation of a-Diazo Esters", J. ORG. CHEM., Bd. 60, 1995, Seiten 1093-1094, XP055311032, Gefunden im Internet: URL:http://pubs.acs.org/doi/pdf/10.1021/jo 00109a053> [gefunden am 2016-10-24]

## Beschreibung

### Gebiet der Erfindung

Die vorliegende Erfindung betrifft Riechstoffmischungen, gemäss anliegender Ansprüche, umfassend mindestens eine Verbindung der allgemeinen Formel (I) 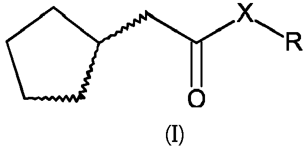 sowie ein Verfahren zur Herstellung von erfindungsgemäßen Riechstoffmischungen, insbesondere Parfümölen, parfümierte Produkte enthaltend die erfindungsgemäße Riechstoffmischung, sowie die Verwendung der Verbindung der allgemeinen Formel (I) zum Verstärken der natürlichen Frische und/oder Ausstrahlung und/oder zum Maskieren oder Vermindern fettiger, und/oder metallischer Noten eines oder mehrerer von der Verbindung der Formel (I) verschiedener Riechstoffe.

### Technologischer Hintergrgund

Grün-krautige und Minzriechstoffe spielen in der Parfümerie eine wichtige Rolle. Es besteht ein ständiger Bedarf, bestimmte geruchliche Aspekte eines Riechstoffes oder einer Riechstoffmischung zu betonen (hervorzuheben), im Falle der genannten Riechstoffe gilt dies insbesondere für deren natürliche Frische und Ausstrahlung. Ebenso besteht ein ständiger Bedarf, bestimmte geruchliche Aspekte eines Riechstoffes oder einer Riechstoffmischung zu maskieren oder zu vermindern, im Falle von den gennannten Riechstoffen gilt dies insbesondere für fettige und metallische Noten.

Die Verbindungen der allgemeinen Formel (I) sind an sich aus dem Stand der Technik bekannt. Geruchsbeschreibungen findet man allerdings nur für bestimmte Verbindungen.

### Relevanter Stand der Technik

Eine Geruchsbeschreibung der Verbindung Cyclopent-2-enyl-essigsäureethylester findet sich in dem Fachbuch "S. Arctander, Perfume and Flavor Materials, Vol. I und II, Montclair, N. J., 1969, Selbstverlag" unter der Nummer 1205. Die Verbindung Cyclopent-2-enyl-essigsäureethylester wird dort als stark, frisch-fruchtig mit einer Note von einer überreifen Ananas beschrieben. Dem Lehrbuch kann ebenfalls entnommen werden, dass die Verbindung in einigen Aromenmischungen eingesetzt wird. Eine Verwendung der Verbindung Cyclopent-2-enyl-essigsäureethylester mit Blumenriechstoffen ist ebenfalls bekannt ( EP 2474301 A1**,** Symrise).

Weiterhin ist aus dem Stand der Technik bekannt, dass Ester der allgemeinen Formel: 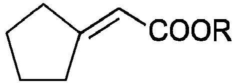 wobei R= Kohlenwasserstoffrest mit C1-C6 C-Atomen: verzweigt, unverzweigt, gesättigt oder ungesättigt bedeutet, einen fruchtigen und blumigen Geruch aufweisen (Patent von Firmenich: DE-PS 2729121**).**

Eine Geruchsbeschreibung einer Verbindung der Formel (A) findet sich im Stand der Technik WO 2009 128026 A1**:**

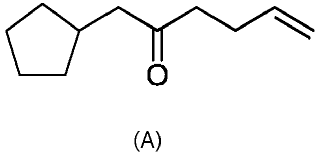

### Aufgabe der Erfindung

Grün-krautige und Minzriechstoffe spielen in der Parfümerie eine wichtige Rolle. Es besteht ein ständiger Bedarf, bestimmte geruchliche Aspekte eines Riechstoffes oder einer Riechstoffmischung zu betonen (hervorzuheben), vor allem deren natürliche Frische und Ausstrahlung. Ebenso besteht ein ständiger Bedarf, bestimmte geruchliche Aspekte eines Riechstoffes oder einer Riechstoffmischung zu maskieren oder zu vermindern, insbesondere fettige und metallische Noten.

Die Aufgabe der vorliegenden Erfindung war es daher, bestimmte geruchliche Aspekte bestimmter Riechstoffe oder Riechstoffmischungen zu betonen bzw. hervorzuheben und/oder bestimmte geruchliche Aspekte eines Riechstoffs oder einer Riechstoffmischung zu maskieren oder zu vermindern, insbesondere fettige und metallische Noten. Weiterhin war es Aufgabe der Erfindung, Riechstoffmischungen zur Verfügung zu stellen, welche die Geruchsnote "grün-krautig", minzig darstellen können und darüber hinaus über positive sekundäre Eigenschaften verfügen.

### Beschreibung der Erfindung

Gelöst wird diese Aufgabe durch Riechstoffmischungen, umfassend mindestens eine Verbindung
(a) der allgemeinen Formel (I): 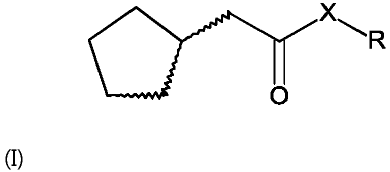 wobei für die Verbindung der Formel (I) bzw. jede Verbindung der Formel (I) gilt, dass X ein Sauerstoffatom oder eine Methylengruppe ist, mit den Maßgaben, dass
   (i) keine oder eine der drei gewellten Linien eine Doppelbindung bedeutet bzw. bedeuten und die übrigen gewellten Linien jeweils eine Einfachbindung darstellen, und
   (ii) R für einen linearen oder verzweigten, cyclischen, gesättigten oder ungesättigten Kohlenwasserstoffrest mit 1 bis 5 Kohlenstoffatomen steht, und
(b) einen oder mehrere weitere Riechstoffe mit einer grün-krautigen und minzigen Geruchsnote enthalten, die ausgewählt sind die gebildet wird von Cantryl, Peonile, Menthon, Frescomenthe, Isooctanon, Tolylacetaldehyd, Phenylacetaldehyd, Cyclogalbanat, Ethylcinnamat oder Mischungen daraus.

In einer bevorzugten Ausführungsform der erfindungsgemäßen Riechstoffmischungen sind die Verbindungen (a) der allgemeinen Formel (I) ausgewählt aus Verbindungen der Formel (Ia) oder (Ib), oder Mischungen daraus 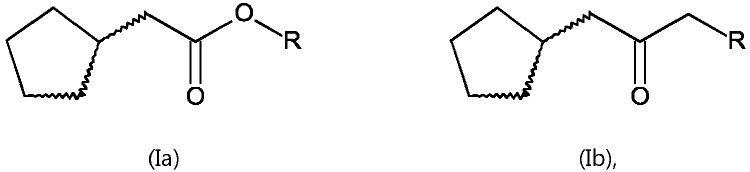 wobei für die Verbindung der Formel (Ia) oder (Ib) bzw. jede Verbindung der Formel (Ia) oder (Ib) gilt, dass
(i) keine oder eine der drei gewellten Linien eine Doppelbindung bedeutet bzw. bedeuten und die übrigen gewellten Linien jeweils eine Einfachbindung darstellen, und
(ii) R für einen linearen oder verzweigten, cyclischen, gesättigten oder ungesättigten Kohlenwasserstoffrest mit 1 bis 5 Kohlenstoffatomen steht.

Vorzugsweise werden bei Mischungen die Verbindungen (Ia) und (Ib) in einem Verhältnis von 1:99 bis 99:1, bevorzugt 20:80 und 40:60, besonders bevorzugt 30:70 und 70:30.eingesetzt.

Verbindungen der allgemeinen Formel (I) und insbesondere der Formel (Ia) und/oder (Ib) weisen dabei Geruchsprofile auf, die in Richtung Apfel, Birne, Ananas, Banane und Erdbeere gehen.

Erfindungsgemäß umfassen die erfindungsgemäßen Riechstoffmischungen ferner einen oder mehrere weitere Riechstoffe (b), insbesondere mit einer grün-krautigen und minzigen Geruchsnote,
- aus der Gruppe bestehend aus Ketonen und Nitrilen mit einer Molmasse von 120 g/mol bis 210 g/mol, bevorzugt 140 g/mol bis 170 g/mol; und/oder
- einen oder mehrere Riechstoffe aus der Gruppe bestehend aus Aldehyden und Estern mit einer Molmasse im Bereich von 190 g/mol bis 250 g/mol,
wobei es sich konkret um einen oder mehrere der folgenden Stoffe handelt: Cantryl, Peonile, Menthon, Frescomenthe, Isooctanon, Tolylacetaldehyd, Phenylacetaldehyd, Cyclogalbanat, Ethylcinnamat

In einer weiteren Ausführungsform, umfassen die erfindungsgemäßen Riechstoffmischungen ferner einen oder mehrere weitere Riechstoffe (c), welche als Fondnote fungieren, ausgewählt aus der Gruppe bestehend aus Ketonen und Estern oder Lactonen mit einer Molmasse im Bereich von 220 g/mol bis 320 g/mol.

Es hat sich vorteilhafterweise herausgestellt, dass Verbindungen der Formel (I), insbesondere Verbindungen der Formel (Ia) und/oder (Ib) zusammen mit Riechstoffen b) und gegebenfalls c) die Geruchsnote "grün-krautig", minzig darstellen können.

In einer solchen erfindungsgemäßen Riechstoffmischung ist das Massenverhältnis der Gesamtmenge an Riechstoffen (b) zur Verbindung(en) (a) vorzugsweise größer oder gleich 99 : 1, bevorzugt größer oder gleich 99,9 : 0,1, besonders bevorzugt größer oder gleich 99,999 : 0,001; bevorzugt 95 : 5; und/oder das Massenverhältnis der Gesamtmenge an Riechstoffen (c) zur Verbindung(en) (a) ist größer oder gleich 99 : 1, bevorzugt größer oder gleich 99,9 : 0,1, besonders bevorzugt größer oder gleich 99,999 : 0,001; jeweils bezogen auf die Gesamtmenge aller Riechstoffe in den einzelnen Verbindungsgruppen (a), (b) und (c), bezogen auf die gesamte Riechstoffmischung.

Ein weiterer Vorteil der erfindungsgemäßen Riechstoffmischung ist ihre hohe Duftintensität bei vergleichsweise geringer Dosierung. Weiterhin wird die "grün-krautig", minzige Note besonders schnell wahrgenommen.

Bevorzugt sind erfindungsgemäße Riechstoffmischungen als Parfümöle.

Ein weiterer Aspekt der vorliegenden Erfindung ist daher die Verwendung der erfindungsgemäßen Riechstoffmischungen als Parfümöl.

Ein weiterer Aspekt der vorliegenden Erfindung ist die Verwendung der erfindungsgemäßen Riechstoffmischungen zur Erzeugung, Vermittlung, Modifizierung oder Verstärkung eines fruchtigen Geruchs in Richtung Apfel, Birne, Ananas, Banane und Erdbeere.

### Herstellung von Verbindungen der Formel (I)

Verbindungen der allgemeinen Formel (I) können z. B. gemäß der DE2729121, EP 2474301 synthetisiert werden oder durch eine Reformatsky-Synthese mit anschließender Wasserabspaltung bzw. durch Veresterung der entsprechenden Säure bzw. Umesterung der entsprechenden Methylestern hergestellt werden:

Zu den gesättigten Verbindungen der allgemeinen Formel (I) gelangt man durch Hydrierung der ungesättigten Ester.

Ebenfalls gelangt man zu Verbindungen der Formel (I) bzw. (Ib) über eine Carroll-Umlagerung oder Claisen-Umlagerung (siehe Formelschema).

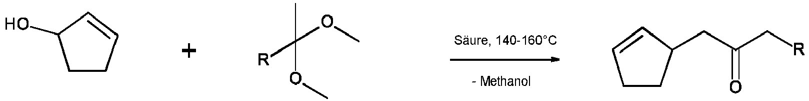

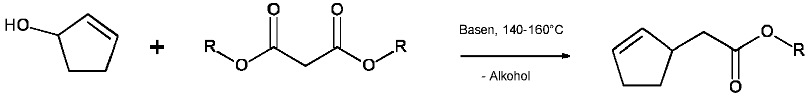

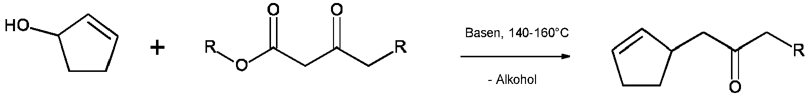

Zu den gesättigten Verbindungen der allgemeinen Formel (Ib) gelangt man durch Hydrierung der ungesättigten Ketone.

### Verbindungen der allgemeinen Formel (I)

Vorzugsweise sind Verbindungen (a) der allgemeinen Formel (I) ausgewählt aus Verbindungen der Formel (Ia) und/oder (Ib).

Hierbei sind die Verbindungen besonders bevorzugt ausgewählt aus der Gruppe bestehend aus:

### Methyl 2-cyclopent-2-en-1-ylacetate (Verbindung II)

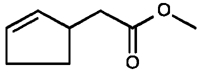

### Ethyl 2-cyclopent-2-en-1-ylacetate (Verbindung III)

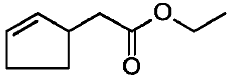

### Propyl 2-cyclopent-2-en-1-ylacetate (Verbindung IV)

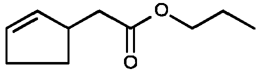

### Isopropyl 2-cyclopent-2-en-1-ylacetate (Verbindung V)

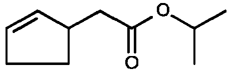

### Allyl 2-cyclopent-2-en-1-ylacetate (Verbindung VI)

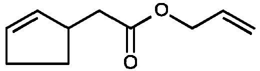

### Isopentyl 2-cyclopent-2-en-1-ylacetate (Verbindung VII)

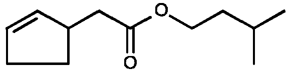

### Cyclopentyl 2-cyclopent-2-en-1-ylacetate (Verbindung VIII)

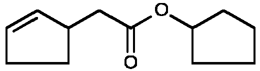

### [(E)-But-2-enyl] 2-cyclopent-2-en-1-ylacetate (Verbindung IX)

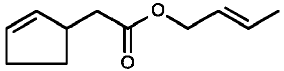

### Cyclopropylmethyl 2-cyclopent-2-en-1-ylacetate (Verbindung X)

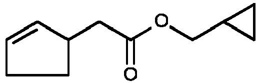

### 1,2-Dimethylpropyl 2-cyclopent-2-en-1-ylacetate (Verbindung XI)

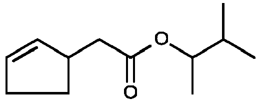

### Ethyl 2-(cyclopenten-1-yl) acetate (Verbindung XII)

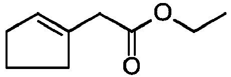

### Methyl 2-cyclopentylideneacetate(Verbindung XIII)

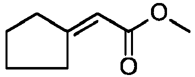

### Methyl 2-cyclopentylacetate (Verbindung XIV)

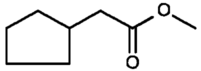

### Propyl 2-cyclopentylacetate (Verbindung XV)

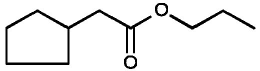

### Allyl 2-cyclopentylacetate (Verbindung XVI)

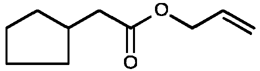

### Isopropyl 2-cyclopentylacetate (Verbindung XVII)

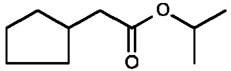

### [(E)-But-2-enyl] 2-cyclopentylacetate (Verbindung XVIII)

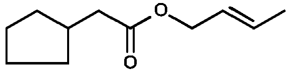

### 3-Methylbut-2-enyl 2-cyclopent-2-en-1-ylacetate (Verbindung XIX)

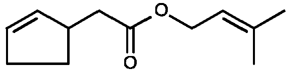

### Ethyl 2-cyclopentylacetate (Verbindung XX)

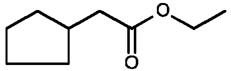

### 1-Cyclopent-2-en-1-ylpropan-2-one (Verbindung XXI)

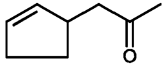

### 1-Cyclopent-2-en-1-ylhexan-2-one (Verbindung XXII)

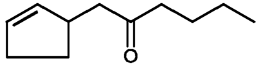

### 1-Cyclopent-2-en-1-ylpent-4-en-2-one (Verbindung XXIII)

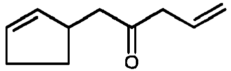

### (E)-1-Cyclopent-2-en-1-ylpent-3-en-2-one (Verbindung XXIV)

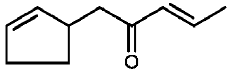

### (Z)-1-Cyclopent-2-en-1-ylpent-3-en-2-one (Verbindung XXV)

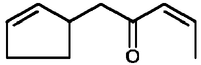

Besonders bevorzugt sind die Verbindungen (Ia) und (Ib) ausgewählt aus den Verbindungen II, VI, VII, XII, XIV, XV, XVI, XX oder XXIII bzw. Mischungen daraus.

### Riechstoffe (b)

Bevorzugt sind Riechstoffe (b) der erfindungsgemäßen Riechstoffmischungen ausgewählt aus der Gruppe bestehend aus Ketonen, Nitrilen mit einer Molmasse von 120-210 g/mol, vorzugsweise 140 g/mol bis 200 g/mol und/oder Aldehyden und Estern mit einer Molmasse von bevorzugt 110 g/mol bis 250 g/mol, bevorzugt 120 g/mol bis 200 g/mol.

Bevorzugt weisen Riechstoffe (b) eine grün-krautige und minzige Geruchsnote auf. Solche Riechstoffe (b) auf Basis von Ketonen, Nitrilen und/oder Aldehyden und Estern sind dem Fachmann bekannt.

Bevorzugt sind erfindungsgemäße Riechstoffmischungen, vorzugsweise Parfümöle, wobei Riechstoffe (b) bevorzugt zwei, drei, vier, fünf oder mehr unterschiedliche Riechstoffverbindungen umfassen.

Vorzugsweise ist in einer erfindungsgemäßen Riechstoffmischung das Massenverhältnis der Gesamtmenge an Riechstoffen (b) zur Verbindung(en) der allgemeinen Formel (I), insbesondere der Formel (Ia) und/oder (Ib) größer oder gleich 99 : 1, bevorzugt größer oder gleich 99,9 : 0,1, besonders bevorzugt größer oder gleich 99,999 : 0,001, jeweils bezogen auf die Gesamtmenge aller Riechstoffe in den einzelnen Verbindungsgruppen (a) und (b), bezogen auf die gesamte Riechstoffmischung.

In Untersuchungen hat sich gezeigt, dass diese Massenverhältnisse besonders vorteilhaft sind, da der Eigengeruch der Verbindung(en) der allgemeinen Formel (I), insbesondere der Formel (Ia) und/oder (Ib) regelmäßig nicht mehr oder kaum noch wahrnehmbar ist, jedoch bewirkt die Anwesenheit der Verbindung(en) der allgemeinen Formel (I) und insbesondere der Formel (Ia) und/oder (Ib) einen positiven Einfluss auf die Gesamtduftnote der erfindungsgemäßen Riechstoffmischung. Besonders überraschend ist, dass die Verbindung(en) der allgemeinen Formel (I), insbesondere der Formel (Ia) und/oder (Ib) auch in geringen Konzentrationen eine Auswirkung auf die Frische und Ausstrahlung der Riechstoffmischung hat, ohne in relevantem Maße einen fruchtigen Geruch zu bewirken oder zu betonen.

Besonders bevorzugt sind erfindungsgemäße Riechstoffmischungen, vorzugsweise erfindungsgemäße Parfümöle, wobei der oder die Riechstoffe (b) ausgewählt sind aus der Gruppe bestehend aus Cantryl, Peonile, Menthon, Frescomenthe, Isooctanon, Tolylacetaldehyd, Phenylacetaldehyd, Cyclogalbanat, Ethylcinnamat oder Mischungen daraus.

Bevorzugt sind erfindungsgemäße Riechstoffmischungen, vorzugsweise erfindungsgemäße Parfümöle, wobei der eine, mehrere oder sämtliche Riechstoffe (b) jeweils eine Molmasse im Bereich von 140 bis 200 g/mol aufweisen

Ganz besonders bevorzugt sind erfindungsgemäße Riechstoffmischungen, vorzugsweise erfindungsgemäße Parfümöle, in deren der Riechstoff (b) einer der angegebenen Stoffe mit Ketonstruktur ist.

Überraschenderweise werden die sensorischen Eigenschaften von Riechstoffen (b) durch Kombination mit einer Menge der Verbindung(en) der allgemeinen Formel (I) und insbesondere der Formel (Ia) und/oder (Ib) positiv beeinflusst. Im Einzelfall wird der sensorische Eindruck in Richtung natürlicher, frischer, mehr Ausstrahlung, weniger fettig und/oder weniger metallisch verschoben, wobei im Einzelfall selbstverständlich auch weitere sensorische Einflüsse zu beobachten waren. Detaillierte Geruchsbeschreibungen finden sich in den beigefügten Beispielen.

### Riechstoffe (c)

Bevorzugt sind Riechstoffe (c) der erfindungsgemäßen Riechstoffmischungen, vorzugsweise Parfümöle, ausgewählt aus der Gruppe bestehend aus Ketonen, Estern/Lactonen und Acetale mit einer Molmasse im Bereich von 220 g/mol bis 320 g/mol.

Bevorzugt sind erfindungsgemäße Riechstoffmischungen, vorzugsweise Parfümöle, wobei Riechstoffe (c) bevorzugt zwei, drei, vier, fünf oder mehr verschiedene Riechstoffverbindungen aufweisen, wie z.B. Globalide, Globanon, Macrolide, Iso E Super, Vertofix, Ambrettolide, Ethylenbrassylat und Ambrocenide.

Die Riechstoffe (c) fungieren als Fondnoten einer erfindungsgemäßen Riechstoffmischung bzw. eines erfindungsgemäßen Parfümöls.

Vorzugsweise ist in einer erfindungsgemäßen Riechstoffmischung das Massenverhältnis der Gesamtmenge an Riechstoffen (c) zur Verbindung(en) der allgemeinen Formel (I), insbesondere der Formel (Ia) und/oder (Ib) größer oder gleich 99 : 1 ist, bevorzugt größer oder gleich 99,9 : 0,1, besonders bevorzugt größer oder gleich 99,999 : 0,001 ist.

In Untersuchungen hat sich gezeigt, dass diese Massenverhältnisse besonders vorteilhaft sind, da der Eigengeruch der Verbindung(en) der allgemeinen Formel (I), insbesondere der Formel (Ia) und/oder (Ib) regelmäßig nicht mehr oder kaum noch wahrnehmbar ist, jedoch bewirkt die Anwesenheit der Verbindung(en) der allgemeinen Formel (I) und insbesondere der Formel (Ia) und/oder (Ib) einen positiven Einfluss auf die Gesamtduftnote der erfindungsgemäßen Riechstoffmischung. Besonders überraschend ist, dass die Verbindung(en) der allgemeinen Formel (I), insbesondere der Formel (Ia) und/oder (Ib) auch in geringen Konzentrationen eine Auswirkung auf die Frische und Ausstrahlung der Riechstoffmischung hat, ohne in relevantem Maße einen fruchtigen Geruch zu bewirken oder zu betonen.

Beispiele für Riechstoffe (c) mit einem Molgewicht im Bereich von 190 g/mol bis 250 g/mol, sind dem Fachmann bekannt und beispielsweise zu finden in S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

Besonders bevorzugt sind erfindungsgemäße Riechstoffmischungen, vorzugsweise erfindungsgemäße Parfümöle, wobei der oder die Riechstoffe (c) ausgewählt sind aus der Gruppe bestehend aus Methyldihydrojasmonat, Benzylsalicylat, cis-3-Hexenylsalicylat, Isoamylsalicylat, Hexylsalicylat, Cedrylacetat, Decahydro-beta-naphthylacetat, 4,7-Methano-3a,4,5,6,7,7a-hexahydro-5(-6)-indenylacetat, Allyl-3-cyclohexylpropionat, Allylcyclohexyloxyacetat, Benzylbenzoat, Benzylcinnamat, Oxacyclopentadecan-2-one, (12E/Z)-1-Oxacyclopentadec-12-en-2-one, (12/13E/Z)-1-Oxacyclohexadec-12/13-en-2-one, Oxacyclohexadecan-2-one, 3-Methyl-cyclopentadecenon, 1,3,4,6,7,8-hexahydro-4,6,6,7,8,8-hexamethyl Cyclopenta[g]-2-benzopyran, 2-[1-(3,3-dimethylcyclohexyl)ethoxy]-2-methyl-,1-propanoat, 1,4-Dioxacycloheptadecan-5,17-dion, 3-Methy-cyclopentadecanon, 8-Cyclohexadecen-1-on, 3a,6,6,9a-Tetramethyl dodecahydronaphtho[2,1-b]furan, alpha-Iron, beta-Iron, alpha-n-Methylionon, beta-n-Methylionon, alpha-Isomethylionon, beta-Isomethylionon und Allylionon.

Überraschenderweise werden die sensorischen Eigenschaften von Riechstoffen (c) durch Kombination mit (einer Menge von) Riechstoffen (b) und Verbindung(en) der allgemeinen Formel (I), insbesondere der Formel (Ia) und/oder (Ib) positiv beeinflusst. Im Einzelfall wird der sensorische Eindruck in Richtung natürlicher, frischer, mehr Ausstrahlung, weniger fettig und/oder weniger metallisch verschoben, wobei im Einzelfall selbstverständlich auch weitere sensorische Einflüsse zu beobachten waren. Detaillierte Geruchsbeschreibungen finden sich in den beigefügten Beispielen.

### Verfahren

Ein weiterer Gegenstand der vorliegenden Erfindung ist ein Verfahren zur Herstellung einer Riechstoffmischung, umfassend die folgenden Schritte:
(a) Bereitstellen einer Verbindung der allgemeinen Formel (I) 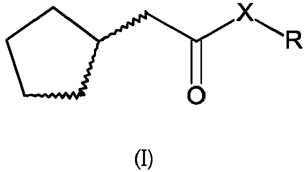 wobei für die Verbindung der Formel (I) bzw. jede Verbindung der Formel (I) gilt, dass X ein Sauerstoffatom oder eine Methylengruppe ist, mit den Maßgaben, dass
   (i) keine oder eine der drei gewellten Linien eine Doppelbindung bedeutet bzw. bedeuten und die übrigen gewellten Linien jeweils eine Einfachbindung darstellen, und
   (ii) R für einen linearen oder verzweigten, cyclischen, gesättigten oder ungesättigten Kohlenwasserstoffrest mit 1 bis 5 Kohlenstoffatomen steht, und
(b) Bereitstellen mindestens eines weiteren Riechstoffs aus der Gruppe, die gebildet wird von Cantryl, Peonile, Menthon, Frescomenthe, Isooctanon, Tolylacetaldehyd, Phenylacetaldehyd, Cyclogalbanat, Ethylcinnamat oder Mischungen daraus, sowie gegebenenfalls
(c) Bereitstellen mindestens eines oder mehrerer weiterer Riechstoffe ausgewählt aus der Gruppe bestehend aus Ketonen und Estern oder Lactonen mit einer Molmasse im Bereich von 220 g/mol bis 320 g/mol, sowie
(d) Vermischen der Bestandteile (a), (b) und gegebenenfalls (c).

### Riechstoffmischungen

Bevorzugt sind Riechstoffischungen umfassend:
(a) Verbindungen VII und XVI, und
(b) Cantryl, Menthon, Phenylacetaldehyd, Ethylcinnamat, und
(c) Methyldihydrojasmonat, Benzylbenzoat, Benzylcinnamat, Oxacyclopentadecan-2-one, (12E/Z)-1-Oxacyclopentadec-12-en-2-one, (12/13E/Z)-1-Oxacyclohexadec-12/13-en-2-one, Oxacyclohexadecan-2-one, 3-Methyl-cyclopentadecenon, 1,3,4,6,7,8-hexahydro-4,6,6,7,8,8-hexamethyl 3-Methy-cyclopentadecanon, 8-Cyclohexadecen-1-on, 3a,6,6,9a-Tetramethyl dodecahydronaphtho[2,1-b]furan, alpha-n-Methylionon, beta-Isomethylionon und Allylionon.

Weiterhin bevorzugt sind Riechstoffischungen umfassend:
(a) Verbindungen II und XXII, und
(b) armanyl, Menthon, Frescomenthe, Phenylacetaldehyd, Cyclogalbanat, und
(c) Methyldihydrojasmonat, Benzylsalicylat, cis-3-Hexenylsalicylat, 4,7-Methano-3a,4,5,6,7,7a-hexahydro-5(-6)-indenylacetat, Oxacyclopentadecan-2-one, (12E/Z)-1-Oxacyclopentadec-12-en-2-one, 2-[1-(3,3-dimethylcyclohexyl)ethoxy]-2-methyl-,1-propanoat, 1,4-Dioxacycloheptadecan-5,17-dion, beta-Isomethylionon und Allylionon.

Eine weitere bevorzugte Riechstoffmischung umfasst
(a) Verbindungen XIV und XXIII, und
(b) Peonile, Isooctanon, Tolylacetaldehyd, Phenylacetaldehyd, und
(c) Allyl-3-cyclohexylpropionat, Allylcyclohexyloxyacetat, Benzylbenzoat, Benzylcinnamat, Oxacyclopentadecan-2-one, (12E/Z)-1-Oxacyclopentadec-12-en-2-one, (12/13E/Z)-1-Oxacyclohexadec-12/13-en-2-one, 2-[1-(3,3-dimethylcyclohexyl)ethoxy]-2-methyl-,1-propanoat, 1,4-Dioxacycloheptadecan-5,17-dion, 3-Methy-cyclopentadecanon, 8-Cyclohexadecen-1-on, 3a,6,6,9a-Tetramethyl dodecahydronaphtho[2,1-b]furan, alpha-Iron, beta-Iron, beta-n-Methylionon, beta-Isomethylionon und Allylionon.

Eine weitere bevorzugte Riechstoffmischung umfasst
(a) Verbindungen XV und XXII, und
(b) Menthon, Isooctanon, Phenylacetaldehyd, und
(c) Methyldihydrojasmonat, Benzylsalicylat, Cedrylacetat, Benzylcinnamat, Oxacyclopentadecan-2-one, 3-Methy-cyclopentadecanon, 8-Cyclohexadecen-1-on, alpha-n-Methylionon.

Eine weitere bevorzugte Riechstoffmischung umfasst
(a) Verbindungen XX und XXI, und
(b) Isooctanon, Tolylacetaldehyd, Phenylacetaldehyd, Ethylcinnamat, und
(c) Benzylsalicylat, Isoamylsalicylat, Hexylsalicylat, Cedrylacetat, Allylcyclohexyloxyacetat, Benzylbenzoat, Oxacyclohexadecan-2-one, 3-Methyl-cyclopentadecenon, 8-Cyclohexadecen-1-on, 3a,6,6,9a-Tetramethyl dodecahydronaphtho[2,1-b]furan, alpha-Iron, beta-Iron, beta-Isomethylionon.

Eine weitere bevorzugte Riechstoffmischung umfasst
(a) Verbindungen VI, XIV, und XX, und
(b) Menthon, Isooctanon, Phenylacetaldehyd, und
(c) Benzylsalicylat, Isoamylsalicylat, Hexylsalicylat, Cedrylacetat, Allylcyclohexyloxyacetat, Benzylbenzoat, Oxacyclohexadecan-2-one.

Diese bevorzugten Riechstoffmischungen können natürlich weitere Riechstoffe umfassen, die nicht unter einer der Riechstoffegruppen (a), (b) und (c) fallen, um die gewünschte Duftnote zu verfeinern.

Erfindungsgemäße Riechstoffmischungen, insbesondere erfindungsgemäße Parfümöle, können in flüssiger Form, unverdünnt oder mit einem Lösungsmittel verdünnt zur Parfümierung oder Aromatisierung eingesetzt werden. Geeignete Lösungsmittel hierfür sind insbesondere Ethanol, Glycerin, 1,2-Propylenglycol, 1,2-Butylenglycol, Dipropylenglycol, Diethylphthalat, Triethylcitrat, Isopropylmyristat und Triacetin.

Bevorzugt werden erfindungsgemäße Riechstoffmischungen, vorzugsweise erfindungsgemäße Parfümöle, mit weiteren Bestandteilen kombiniert. Bevorzugte weitere Bestandteile sind ausgewählt aus der Gruppe bestehend aus:

Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungs¬hemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildnder, vorzugsweise die in WO 2005/123101 genannten, Zellstimulantien, reinigende Mittel, pflegende Mittel, Enthaarungsmittel, ober¬flächen¬aktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, Fixateure, Schaum¬bildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtig¬keitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-beta-dicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Wasch-,mittel, Weichspüler, suspendierende Mittel, Hautbräunungsmittel, vorzugsweise die in WO 2006/045760 genannten, Ver¬dickungs¬mittel, Vitamine, vorzugsweise die in WO 2005/123101 genannten, Öle, Wachse und Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxy¬fettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, Aromen und Geschmackstoffe sowie weitere zusätzliche Riechstoffe, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, insbesondere die in US 2008/0070825 explizit genannten weiteren Riechstoffe, die nicht bereits Teil der Bestandteile (b) sowie (c) einer erfindungsgemäßen Riechstoffmischung bzw. eines erfindungsgemäßen Parfümöls sind, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, oder Silikone und Silikonderivate, vorzugsweise die in WO 2008/046676 genannten. Verbindungen, die unter die Definition der Bestandteile (b) und/oder (c) fallen, werden allerdings unabhängig vom Einsatzzweck diesen Bestandteilen zugeordnet; zu Ausnahmen für bestimmte Lösungsmittel siehe oben.

### Träger und Kapseln

Des weiteren können erfindungsgemäße Riechstoffmischungen, insbesondere erfindungsgemäße Parfümöle, an einem Trägerstoff adsorbiert sein, der sowohl für eine feine Verteilung der darin enthaltenen Riechstoffe im Produkt als auch für eine kontrollierte Freisetzung bei der Anwendung sorgt. Derartige Träger können poröse anorganische Materialien wie Leichtsulfat, Kieselgele, Zeolithe, Gipse, Tone, Ton-granulate, Gasbeton usw. oder organische Materialien wie Hölzer, Cellulose-basierende Stoffe, Zucker, Dextrine (z.B. Maltodextrin) oder Kunststoffe wie PVC, Polyvinylacetate oder Polyurethane sein. Die Kombination aus erfindungsgemäßer Riechstoffmischung und Trägerstoff stellt ein beispielhaftes erfindungsgemäßes Produkt dar.

Erfindungsgemäße Riechstoffmischungen, insbesondere erfindungsgemäße Parfümöle können auch mikroverkapselt, sprühgetrocknet, als Einschluss-Komplexe oder als Extrusions-Produkte (d. h. erfindungsgemäße Produkte) vorliegen und in dieser Form z.B. einem zu parfümierenden Produkt hinzugefügt werden.

Gegebenenfalls können die Eigenschaften der derart modifizierten Kompositionen durch sog. "Coaten" mit geeigneten Materialien im Hinblick auf eine gezieltere Duftfreisetzung weiter optimiert werden, wozu vorzugsweise wachsartige Kunststoffe wie z.B. Polyvinylalkohol verwendet werden. Die resultierenden Produkte stellen wiederum erfindungsgemäße Produkte dar.

Die Mikroverkapselung der erfindungsgemäßen Riechstoffmischungen, vorzugsweise der erfindungsgemäßen Parfümöle, kann beispielsweise durch das sogenannte Koazervationsverfahren mit Hilfe von Kapselmaterialien z.B. aus polyurethanartigen Stoffen oder Weichgelatine, erfolgen. Die sprühgetrockneten Riech- oder Aromastoffkompositionen können beispielsweise durch Sprühtrocknung einer die erfindungsgemäße Riechstoffmischung, vorzugsweise ein Parfümöl, enthaltenden Emulsion, bzw. Dispersion hergestellt werden, wobei als Trägerstoffe modifizierte Stärken, Proteine, Dextrin und pflanzliche Gummen verwendet werden können. Einschluss-Komplexe können z.B. durch Eintragen von Dispersionen der erfindungsgemäßen Riechstoffmischung, vorzugsweise einem erfindungsgemäßen Parfümöl, und Cyclodextrinen oder Harnstoffderivaten in ein geeignetes Lösungsmittel, z.B. Wasser, hergestellt werden. Extrusions-Produkte können durch Verschmelzen einer erfindungsgemäßen Riechstoffmischung, vorzugsweise eines erfindungsgemäßen Parfümöls, mit einem geeigneten wachsartigen Stoff und durch Extrusion mit nachfolgender Erstarrung, ggf. in einem geeigneten Lösungsmittel, z.B. Isopropanol, erhalten werden.

Überraschenderweise bewirken Verbindungen der Formel (I), insbesondere Verbindung(en) (Ia) und/oder (Ib) in Riechstoffmischungen mit Riechstoffen (b), dass bestimmte geruchliche Aspekte des oder der Riechstoffe (b), betont bzw. hervorgehoben und/oder maskiert bzw. vermindert werden. Insbesondere fettige und metallische Noten der Riechstoffe der Komponenten (b) werden durch Verbindungen der Formel (I), insbesondere Verbindung(en) (Ia) und/oder (Ib) effektiv maskiert bzw. vermindert.

Riechstoffe (b), weisen, wie vorstehend bereits beschrieben, vorzugsweise eine grün-krautige und minzige Geruchsnote auf, und sind ausgewählt aus der Gruppe bestehend aus Ketonen und Nitrilen mit einer Molmasse von 120 g/mol bis 210 g/mol, bevorzugt 140g/mol bis 170 g/mol; und/oder einen oder mehrere Riechstoffe aus der Gruppe bestehend aus Aldehyden und Estern mit einer Molmasse im Bereich von 190 g/mol bis 250 g/mol.

Daher ist ein weiterer Aspekt der vorliegenden Erfindung ein Verfahren zum Verstärken der natürlichen Frische und/oder Ausstrahlung und/oder zum Maskieren oder Vermindern fettiger und/oder metallischer Noten eines oder mehrerer Verbindung(en) der allgemeinen Formel (I) verschiedener Riechstoffe, insbesondere der von Formel (Ia) und/oder (Ib) verschiedener Riechstoffe, mit einer grün-krautigen und/oder minzigen Geruchsnote umfassend den folgenden Schritt:
Vermischen des oder der von Verbindung(en) der allgemeinen Formel (I), insbesondere der von Formel (Ia) und/oder (Ib) verschiedenen Riechstoffe mit einer Menge an Verbindung der Formel (I), insbesondere (Ia) und/oder (Ib), die ausreicht, die natürliche Frische und/oder Ausstrahlung des oder der von Verbindung(en) der allgemeinen Formel (I) verschiedener Riechstoffe, insbesondere der von Formel (Ia) und/oder (Ib) verschiedener Riechstoffe, zu verstärken und/oder fettige und/oder metallische Noten zu maskieren oder zu vermindern.

Im vorliegenden erfindungsgemäßen Verfahren ist bzw. sind der oder die von der Verbindung der Formel (I) verschiedenen Riechstoffe, insbesondere der von Formel (Ia) und/oder (Ib) verschiedener Riechstoffe, ausgewählt aus
b) mindestens einen weiteren Riechstoff, vorzugsweise mit einer grün-krautigen und minzigen Geruchsnote, aus der Gruppe bestehend aus Ketonen und Nitrilen mit einer Molmasse von 120 g/mol bis 210 g/mol, bevorzugt 140 g/mol bis 170 g/mol;
und/oder
einen oder mehreren Riechstoffe aus der Gruppe bestehend aus Aldehyden und Estern mit einer Molmasse im Bereich von 190 g/mol bis 250 g/mol
und /oder
c) einen oder mehreren weiteren Riechstoffe ausgewählt aus der Gruppe bestehend aus Ketonen und Estern oder Lactonen mit einer Molmasse im Bereich von 220 g/mol bis 320 g/mol.

Das Massenverhältnis der Gesamtmenge an Riechstoffen (b) zur Verbindung(en) (a) ist hierbei größer oder gleich 99 : 1, bevorzugt größer oder gleich 99,9 : 0,1, besonders bevorzugt größer oder gleich 99,999 : 0,001;
und/oder
das Massenverhältnis der Gesamtmenge an Riechstoffen (c) zur Verbindung(en) (a) ist größer oder gleich 99 : 1, bevorzugt größer oder gleich 99,9 : 0,1, besonders bevorzugt größer oder gleich 99,999 : 0,001;
jeweils bezogen auf die Gesamtmenge aller Riechstoffe in den einzelnen Verbindungsgruppen (a), (b) und (c), bezogen auf die gesamte Riechstoffmischung.

Überraschenderweise hat sich gezeigt, dass die Verbindungen der Formel (I), insbesondere Verbindunge (Ia) und/oder (Ib) über ihre primären sensorischen Eigenschaften hinaus zusätzliche positive Sekundäreigenschaften, wie z.B. eine hohe Stabilität unter bestimmten Anwendungsbedingungen (in alkalischen Medien (Waschpulver, Wäscheweich, Seife, Shampoo etc.), eine hohe Ausgiebigkeit, ein gutes Haftungsvermögen, eine hohe Substantivität aufweisen.

Demgemäß ist ein weiterer Gegenstand der vorliegenden Erfindung ein parfümiertes Produkt enthaltend eine erfindungsgemäße Riechstoffmischung, vorzugsweise ein erfindungsgemäßes Parfümöl, wie vorstehend beschrieben, in einer sensorisch wirksamen Menge.

"Sensorisch wirksame Menge" bedeutet im vorliegenden Zusammenhang, dass das erfindungsgemäße parfümierte Produkt im Betrieb bzw. bei Benutzung die sensorischen Eigenschaften der erfindungsgemäßen Riechstoffmischung erkennen lässt.

Der Anteil der Riechstoffmischung an dem parfümierten Produkt in einem erfindungsgemäßen parfümierten Produkt beträgt vorzugsweise 0,01 bis 10 Gew.-%, bezogen auf die Gesamtmasse des parfümierten Produktes

Bevorzugte erfindungsgemäße parfümierte Produkte sind vorzugsweise ausgewählt aus der Gruppe bestehend aus:
Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und - lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

Besonders bevorzugte erfindungsgemäße parfümierte Produkte sind ausgewählt aus folgender Liste:
- Eau de Parfums, Eau de Toilettes, Rasierwässer (After-shave), Eau de Colognes, Preshave-Produkte, Splash-Colognes;
- saure, alkalische und neutrale Reinigungsmittel, insbesondere im Haushaltsbereich, vorzugsweise Fußbodenreiniger, Fensterglasreiniger, Geschirrspülmittel, Bad- und Sanitärreiniger, Scheuermilch, feste und flüssige WC-Reiniger, pulver- und schaumförmige Teppichreiniger, flüssige Waschmittel, pulverförmige Waschmittel, Wäscheweichspüler, Oberflächendesinfektionsmittel, insbesondere für harte Oberflächen (hard surface cleaner);
- Körperpflegemittel, vorzugsweise feste und flüssige Seifen, Duschgele, Shampoos, Rasierseifen, Rasierschäume;
- kosmetische Emulsionen vom ÖI-in-Wasser-, vom Wasser-in-ÖI- und vom Wasser-in-ÖI-in-Wasser-Typ, vorzugsweise Hautcremes- und -lotionen, Gesichtscremes und - lotionen, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Hautbräunungscremes und -lotionen, Hautaufhellungscremes und -lotionen;
- Haarpflegeprodukte, vorzugsweise Haarsprays, Haargele, festigende Haarlotionen, Haarspülungen, permanente und semipermanente Haarfärbemittel, Haarwässer, Haarcremes und -lotionen;
- Deodorantien und Antiperspirantien, vorzugsweise Achselsprays, Roll-ons (vorzugsweise als alkoholische oder nicht-alkoholische Lösung, als Gel oder (Micro)Emulsion, Deosticks (Deo-stifte), Deocremes.

Besonders bevorzugte erfindungsgemäße parfümierte Produkte sind Wasch- und Reinigungsmittel, Hygiene- oder Pflegeprodukte, insbesondere im Bereich der Körper- und Haarpflege, der Kosmetik und des Haushalts.

Bevorzugte erfindungsgemäße parfümierte Produkte sind solche, bei denen der Anteil der erfindungsgemäßen Riechstoffmischung an dem parfümierten Produkt 0,01 bis 10 Gew.-%, bevorzugt 0,1 bis 5 Gew.-% und besonders bevorzugt 0,25 bis 3 Gew.-% beträgt, jeweils bezogen auf die Gesamtmasse des parfümierten Produktes. Dies gilt insbesondere für die vorstehend genannten bevorzugten Produkte.

Offenbart wird ferner auch ein Verfahren zur Herstellung eines parfümierten Produktes umfassend die Schritte:
i) Bereitstellen einer erfindungsgemäßen Riechstoffmischung oder Herstellen einer Riechstoffmischung nach einem erfindungsgemäßen Verfahren,
ii) Bereitstellen der weiteren Bestandteile des parfümierten Produktes und
iii) Inkontaktbringen der in Schritt ii) bereitgestellten weiteren Bestandteile des parfümierten Produkts mit einer sensorisch wirksamen Menge der in Schritt i) bereitgestellten Riechstoffmischung; wobei die Menge der Verbindung der allgemeinen Formel (I), insbesondere Formel (Ia) und/oder (Ib) ausreicht, um die natürliche Frische und/oder Ausstrahlung eines, mehrerer oder sämtlicher Riechstoffe (b) und/oder (c) zu verstärken und/oder fettige, technische und/oder metallische Noten zu maskieren oder zu vermindern
oder
I) Bereitstellen der Bestandteile des parfümierten Produktes, die keine Riechstoffe (a), (b) bzw. (c) einer erfindungsgemäßen Riechstoffmischung sind
II) Mischen der in Schritt I) bereitgestellten Bestandteile des parfümierten Produkts mit Riechstoffe (b) und/oder (c) einer erfindungsgemäßen Riechstoffmischung, sodass eine Mischung resultiert, in welcher der oder die Riechstoffe (b) und/oder (c) in einer sensorisch wirksamen Menge vorliegen,
III) Inkontaktbringen der in Schritt II) hergestellten Mischung mit einer Menge der Verbindung der Formel (I) bzw. (Ia), wobei die Menge der Verbindung der allgemeinen Formel (I), insbesondere Formel (Ia) und/oder (Ib) ausreicht, um die natürliche Frische und/oder Ausstrahlung eines, mehrerer oder sämtlicher Riechstoffe der Riechstoffe (b) und/oder (c) zu verstärken und/oder fettige, technische und/oder metallische Noten zu maskieren oder zu vermindern.

Bevorzugt werden die erfindungsgemäßen Riechstoffmischungen als Parfümöle, in pharmazeutischen Mitteln oder in kosmetischen Produkten verwendet.

Bevorzugt sind hierbei Anwendungen auf der Haut geeignet sind. Bevorzugt sind hierbei sowohl kosmetische Produkte als auch pharmazeutische Mittel in Form von Salben, Cremes, Lotionen, Gele und Pasten und Sprays.

Vorzugsweise ist unter eine Salbe, Creme, Lotion, Gel und Paste eine halbfeste streichfähige Zubereitung zu verstehen, die zum Auftragen auf die Haut geeignet sind.

Solche Zubereitungen können beispielsweise auf Basis von einer wässrigen (hydrophilen) und einer öligen bzw. fetten (lipophilen) Komponente bestehen, von der die eine emulsionsartig in der anderen verteilt ist.

Es können ebenfalls hydrophile Cremes vom O/W-Typ oder lipophile Cremes vom W/O-Typ sein. Daneben gibt es Cremes, die weder eindeutig dem O/W- noch dem W/O-Typ zuordenbar sind, die aus gelartig, kohärent ineinander verteilter lipophiler und hydrophiler Phase bestehen (amphiphile Creme). Auch Strukturen einer Mehrfachemulsion vom Typ W/O/W-Emulsion sind möglich. Hier liegt die innere Phase wiederum in Form einer Emulsion vor. In die innere Ölphase sind nochmals kleinste Wassertröpfchen eingelagert. Dieser Emulsionstyp soll die Vorteile von W/O-Emulsionen und O/W-Emulsionen in sich vereinen.

Weitere Zubereitungen sind bevorzugt Salben, die in der Regel eine halbfeste und homogen aussehende Zubereitung ist, und die geeigent ist zur Anwendung auf der Haut (z. B. als Wundsalbe) oder auf den Schleimhäuten. Salben dienen zumeist der lokalen Wirkstoffapplikation oder der Pflege und dem Schutz der Haut oder Schleimhäute.

Vorzugsweise besteht eine Salbe aus einer hydrophoben oder hydrophilen Grundlage aus natürlichen oder synthetischen Stoffen und kann ein einphasiges (z. B. Vaseline) oder mehrphasiges (z.B. Wasser-in-ÖI) System sein.

Eine weitere bevorzugte Zubereitung ist das Gel. Gele können als viskoelastische Fluide beschrieben werden. Ihre Fluideigenschaften liegen somit zwischen denen einer idealen Flüssigkeit und denen eines idealen Feststoffkörpers. Ein Gel ist ein feindisperses System aus mindestens einer festen und einer flüssigen Phase. Die feste Phase bildet dabei ein schwammartiges, dreidimensionales Netzwerk, dessen Poren durch eine Flüssigkeit (Lyogel) oder ein Gas (Xerogel) ausgefüllt sind. Ist das Netzwerk hochporös und Luft das eingelagerte Gas, so wird das Gel auch als Aerogel bezeichnet. Beide Phasen durchdringen sich dabei vollständig (bikohärent).

Bevorzugt sind ebenfalls Zubereitungen in Form von Pasten. Eine Paste ist ein Feststoff-Flüssigkeitsgemisch (Suspension) mit einem hohen Gehalt an Festkörpern. Pasten sind vorzugsweise nicht mehr fließfähig, sondern streichfest. Beispielsweise ist eine Paste eine Salbe-Pulver-Mischung und insbesondere halbfeste Arzneiform mit hohem Gehalt an dispergierten Feststoffen ("Suspensionssalbe" mit einem Feststoffanteil von 30 Gew.%-zum Beispiel ist eine solche Paste die Pasta Zinci (Zinksalbe)).

Ein Beispiel, bei dem ein Produkt in den unterschiedlichen Zubereitungsformen vorliegen kann, ist beispielsweise die Zahnpasta (oder- creme oder -gel), die sowohl im medizinischen als auch im kosmetischen Bereich angewendet werden kann, und somit eine breite Produktpalette ist. Der Hauptbestandteil sind Putzkörper, Schaumbildner, Netz- und Feuchthaltemittel, Geschmacks- und Aromastoffe, Konservierungsmittel sowie Farb- und Zusatzstoffe. Außerdem enthalten Zahncremes auch Wirkstoffe zur zahnmedizinischen Prophylaxe, speziell von Parodontitis und Karies (Fluoride).

Erfindungsgemäß können in den oben beschriebenen Zubereitungen (Salben, Cremes, Lotionen, Gele und Pasten) Wirkstoffe und Wirkstoffsubstanzen besonders gut durch die erfindungsgemäßen Kaspeln eingearbeitet werden, da die Kapseln wasserunslöslich und so in der Grundzubereitung stabil sind und erst durch die Anwendung, vorzugsweise durch mechanische Scherung, wie beispielsweise durch das Verreiben auf der Haut oder Putzen von Zähnen, zerstört werden und die Wirkstoffe bzw. Wirksubstanzen freisetzen bzw. abgeben.

Die die erfindungsgemäßen Riechstoffmischungen, bevorzugt Parfümöle, lassen sich demgemäß entsprechend in unterschiedlichen Produkten herstellen.

Ein weiterer Gegenstand der vorliegenden Erfindung sind daher Körperpflegeprodukte, Wasch- und Reinigungsmittel oder Parfümzusammensetzungen, enthaltend die erfindungsgemäßen Riechstoffmischungen, vorzugsweise Parfümöle.

Kosmetische Inhaltstoffe und Wirkstoffe, die in den Körperpflegeprodukten, Wasch- und Reinigungsmitteln oder Parfümzusammensetzungen, enthaltend die erfindungsgemäßen Riechstoffmischungen, vorzugsweise Parfümöle verwendet werden sind im Folgenden aufgelistet. Bevorzugt sind hierbei die Parfümöle, Aromen, Aromastoffe, Duftstoffe.

### Kosmetische Produkte

Vorzugsweise umfassen kosmetische Produkte und pharmazeutische Zubereitungen eine Reihe von Hilfs- und Zusatzstoffe. Diese Hilfs- und Zusatzstoffe können, je nach Notwendigkeit, auch in verkapselt vorliegen. Die typischen Hilfs- und Zusatzstoffe, die in kosmetischen Produkte und/oder pharmazeutischen Mittel enthalten sein können sind beispielsweise milde Tenside, Ölkörper, Emulgatoren, Perlglanzwachse, Kühlstoffe, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen.

### Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, vezweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv^{®} TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### Emulgatoren

Als Emulgatoren kommen beispielsweise nicht ionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia^{®} SP von Cognis;
- Polyalkylenglycole sowie
- Glycerincarbonat.

Im Folgenden werden besonders geeignete Emulgatoren näher erläutert:
**Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Subtrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.

**Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

**Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.

**Sorbitanester.** Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitandimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.

**Polyglycerinester.** Typische Beispiele für geeignete Polyglycerinester sind Polyglyceryl-2-Dipolyhydroxystearate (Dehymuls^{®} PGPH), Polyglycerin-3-Diisostearate (Lameform^{®} TGI), Polyglyceryl-4 Isostearate (Isolan^{®} GI 34), Polyglyceryl-3 Oleate, Diisostearoyl Polyglyceryl-3 Diisostearate (Isolan^{®} PDI), Polyglyceryl-3 Methylglucose Distearate (Tego Care^{®} 450), Polyglyceryl-3 Beeswax (Cera Bellina^{®}), Polyglyceryl-4 Caprate (Polyglycerol Caprate T2010/90), Polyglyceryl-3 Cetyl Ether (Chimexane^{®} NL), Polyglyceryl-3 Distearate (Cremophor^{®} GS 32) und Polyglyceryl Polyricinoleate (Admul^{®} WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.

**Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.

**Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C- Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine - COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxy-substituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### Physiologische Kühlstoffe

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

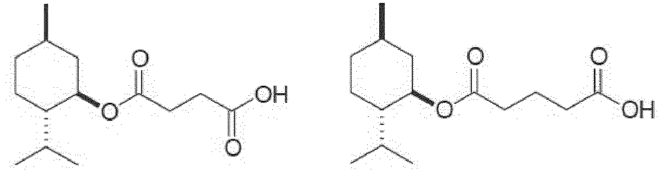

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat^{®} MGC), Menthol Propylenglycol Carbonate (FEMA GRAS 3784 = Frescolat^{®} MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat^{®} ML) und insbesondere das Menthone Glyceryl Acetal (FEMA GRAS 3807) bzw. Menthone Glyceryl Ketal (FEMA GRAS 3808), das unter der Bezeichnung Frescolat^{®} MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat^{®} MGA, Frescolat^{®} ML, Frecolat^{®} MGC und Frescolat^{®} MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und -diester von Fettsäuren, Polyacrylate, (z.B. Carbopole^{®} und Pemulen-Typen von Goodrich; Synthalene^{®} von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium- und/oder Zinkstearat bzw. -ricinoleat eingesetzt werden.

### UV-Lichtschutzfilter

Unter UV-Lichtschutzfilter sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfilter in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoe-säureamylester;
- Ester der Zimtsäure, vorzugsweise 4-Methoxyzimtsäure-2-ethylhexylester, 4-Methoxyzimtsäurepropylester, 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-iso-propylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexyl-ester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb^{®} HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan^{®} AP)
- Sulfonsäurederivate von Benzophenonen, vorzugsweise 2-Hydroxy-4-methoxybenzo-phenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol^{®} 1789), 2-(4-Diethylamino-2-hydroxybenzoyl)-benzoic acid hexylester (Uvinul^{®} A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans" z.B. 4-tert.-Butyl-4'-methoxydibenzoylmethan (Parsol^{®} 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000, Eusolex^{®} T, Eusolex^{®} T-ECO, Eusolex^{®} T-S, Eusolex^{®} T-Aqua, Eusolex^{®} T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE^{®} oder Z-COTE HP1^{®} verwendet.

### Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### Deodorantien und keimhemmende Mittel

Kosmetische Deodorantien (Desodorantien) wirken Körpergerüchen entgegen, überdecken oder beseitigen sie. Körpergerüche entstehen durch die Einwirkung von Hautbakterien auf apokrinen Schweiß, wobei unangenehm riechende Abbauprodukte gebildet werden. Dementsprechend enthalten Deodorantien Wirkstoffe, die als keimhemmende Mittel, Enzyminhibitoren, Geruchsabsorber oder Geruchsüberdecker fungieren.

**Keimhemmende Mittel.** Als keimhemmende Mittel sind grundsätzlich alle gegen grampositive Bakterien wirksamen Stoffe geeignet, wie z. B. 4-Hydroxybenzoesäure und ihre Salze und Ester, N-(4-Chlorphenyl)-N'-(3,4-dichlorphenyl)harnstoff, 2,4,4'-Trichlor-2'-hydroxy-diphenylether (Triclosan), 4-Chlor-3,5-dimethyl-phenol, 2,2'-Methylen-bis(6-brom-4-chlorphenol), 3-Methyl-4-(1-methylethyl)-phenol, 2-Benzyl-4-chlorphenol, 3-(4-Chlorphenoxy)-1,2-propandiol, 3-Iod-2-propinylbutylcarbamat, Chlorhexidin, 3,4,4'-Trichlorcarbanilid (TTC), antibakterielle Riechstoffe, Thymol, Thymianöl, Eugenol, Nelkenöl, Menthol, Minzöl, Farnesol, Phenoxyethanol, Glycerinmonocaprinat, Glycerinmonocaprylat, Glycerinmonolaurat (GML), Diglycerinmonocaprinat (DMC), Salicylsäure-N-alkylamide wie z. B. Salicylsäure-n-octylamid oder Salicylsäure-n-decylamid.

**Enzyminhibitoren.** Als Enzyminhibitoren sind beispielsweise Esteraseinhibitoren geeignet. Hierbei handelt es sich vorzugsweise um Trialkylcitrate wie Trimethylcitrat, Tripropylcitrat, Triisopropylcitrat, Tributylcitrat und insbesondere Triethylcitrat (Hydagen^{®} CAT). Die Stoffe inhibieren die Enzymaktivität und reduzieren dadurch die Geruchsbildung. Weitere Stoffe, die als Esteraseinhibitoren in Betracht kommen, sind Sterolsulfate oder -phosphate, wie beispielsweise Lanosterin-, Cholesterin-, Campesterin-, Stigmasterin- und Sitosterinsulfat bzw -phosphat, Dicarbonsäuren und deren Ester, wie beispielsweise Glutarsäure, Glutarsäuremonoethylester, Glutarsäurediethylester, Adipinsäure, Adipinsäuremonoethylester, Adipinsäurediethylester, Malonsäure und Malonsäurediethylester, Hydroxycarbonsäuren und deren Ester wie beispielsweise Citronensäure, Äpfelsäure, Weinsäure oder Weinsäurediethylester, sowie Zinkglycinat.

**Geruchsabsorber.** Als Geruchsabsorber eignen sich Stoffe, die geruchsbildende Verbindungen aufnehmen und weitgehend festhalten können. Sie senken den Partialdruck der einzelnen Komponenten und verringern so auch ihre Ausbreitungsgeschwindigkeit. Wichtig ist, daß dabei Parfums unbeeinträchtigt bleiben müssen. Geruchsabsorber haben keine Wirksamkeit gegen Bakterien. Sie enthalten beispielsweise als Hauptbestandteil ein komplexes Zinksalz der Ricinolsäure oder spezielle, weitgehend geruchsneutrale Duftstoffe, die dem Fachmann als "Fixateure" bekannt sind, wie z. B. Extrakte von Labdanum bzw. Styrax oder bestimmte Abietinsäurederivate. Als Geruchsüberdecker fungieren Riechstoffe oder Parfümöle, die zusätzlich zu ihrer Funktion als Geruchsüberdecker den Deodorantien ihre jeweilige Duftnote verleihen. Als Parfümöle seien beispielsweise genannt Gemische aus natürlichen und synthetischen Riechstoffen. Natürliche Riechstoffe sind Extrakte von Blüten, Stengeln und Blättern, Früchten, Fruchtschalen, Wurzeln, Hölzern, Kräutern und Gräsern, Nadeln und Zweigen sowie Harzen und Balsamen. Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, p-tert.-Butylcyclohexylacetat, Linalylacetat, Phenylethylacetat, Linalylbenzoat, Benzylformiat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labdanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilat, Irotyl und Floramat allein oder in Mischungen, eingesetzt.

**Antitranspirantien.** Antitranspirantien (Antiperspirantien) reduzieren durch Beeinflussung der Aktivität der ekkrinen Schweißdrüsen die Schweißbildung, und wirken somit Achselnässe und Körpergeruch entgegen. Wässrige oder wasserfreie Formulierungen von Antitranspirantien enthalten typischerweise folgende Inhaltsstoffe:
- adstringierende Wirkstoffe,
- Ölkomponenten,
- nichtionische Emulgatoren,
- Coemulgatoren,
- Konsistenzgeber,
- Hilfsstoffe wie z. B. Verdicker oder Komplexierungsmittel und/oder
- nichtwässrige Lösungsmittel wie z. B. Ethanol, Propylenglykol und/oder Glycerin.

Als adstringierende Antitranspirant-Wirkstoffe eignen sich vor allem Salze des Aluminiums, Zirkoniums oder des Zinks. Solche geeigneten antihydrotisch wirksamen Wirkstoffe sind z.B. Aluminiumchlorid, Aluminiumchlorhydrat, Aluminiumdichlorhydrat, Aluminiumsesquichlorhydrat und deren Komplexverbindungen z. B. mit Propylenglycol-1,2. Aluminiumhydroxyallantoinat, Aluminiumchloridtartrat, Aluminium-Zirkonium-Trichloro-hydrat, Aluminium-Zirkonium-tetrachlorohydrat, Aluminium-Zirkonium-pentachlorohydrat und deren Komplexverbindungen z. B. mit Aminosäuren wie Glycin. Daneben können in Antitranspirantien übliche öllösliche und wasserlösliche Hilfsmittel in geringeren Mengen enthalten sein. Solche öllöslichen Hilfsmittel können z.B. sein:
- entzündungshemmende, hautschützende oder wohlriechende ätherische Öle,
- synthetische hautschützende Wirkstoffe und/oder
- öllösliche Parfümöle.

Übliche wasserlösliche Zusätze sind z.B. Konservierungsmittel, wasserlösliche Duftstoffe, pH-Wert-Stellmittel, z.B. Puffergemische, wasserlösliche Verdickungsmittel, z.B. wasserlösliche natürliche oder synthetische Polymere wie z.B. Xanthan-Gum, Hydroxyethylcellulose, Polyvinylpyrrolidon oder hochmolekulare Polyethylenoxide.

### Antischuppenwirkstoffe

Als Antischuppenwirkstoffe kommen Pirocton Olamin (1-Hydroxy-4-methyl-6-(2,4,4-trimythylpentyl)-2-(1H)-pyridinonmonoethanolaminsalz), Baypival^{®} (Climbazole), Ketoconazol^{®}, (4-Acetyl-1-{-4-[2-(2.4-dichlorphenyl) r-2-(1H-imidazol-1-ylmethyl)-1,3-dioxylan-c-4-ylmethoxyphenyl}piperazin, Ketoconazol, Elubiol, Selendisulfid, Schwefel kolloidal, Schwefelpolyehtylenglykolsorbitanmonooleat, Schwefelrizinolpolyehtoxylat, Schwfel-teer Destillate, Salicylsäure (bzw. in Kombination mit Hexachlorophen), Undexylensäure Monoethanolamid Sulfosuccinat Na-Salz, Lamepon^{®} UD (Protein-Undecylensäurekondensat), Zinkpyrithion, Aluminiumpyrithion und Magnesiumpyrithion / Dipyrithion-Magnesiumsulfat in Frage.

### Insektenrepellentien

Als Insekten-Repellentien kommen N,N-Diethyl-m-toluamid, 1,2-Pentandiol oder Ethyl Butylacetylaminopropionate in Frage. Als Selbstbräuner eignet sich Dihydroxyaceton. Als Tyrosinhinbitoren, die die Bildung von Melanin verhindern und Anwendung in Depigmentierungsmitteln finden, kommen beispielsweise Arbutin, Ferulasäure, Kojisäure, Cumarinsäure und Ascorbinsäure (Vitamin C) in Frage.

### Inhaltsstoffe für Mund- und Zahnpflegemittel

Unter Mund- und Zahnpflegemittel sind Produkte zu verstehen, die der Mund- und Zahnreinigung und-pflege dienen. Beispiele hierfür sind Zahnpasten, Zahngele, und dergleichen.

Unter Zahnpasten oder Zahncremes werden im allgemeinen gelförmige oder pastöse Zubereitungen aus Wasser, Verdickungsmitteln, Feuchthaltemitteln, Schleif- oder Putzkörpern, Tensiden, Süßmitteln, Aromastoffen, deodorierenden Wirkstoffen sowie Wirkstoffen gegen Mund- und Zahnerkrankungen verstanden. In die erfindungsgemäßen Zahnpasten können alle üblichen Putzkörper, wie z. B. Kreide, Dicalciumphosphat, unlösliches Natriummetaphosphat, Aluminiumsilikate, Calciumpyrophosphat, feinteilige Kunstharze, Kieselsäuren, Aluminiumoxid und Aluminiumoxidtrihydrat eingesetzt werden.

Bevorzugt geeignete Putzkörper für die erfindungsgemäßen Zahnpasten sind vor allem feinteilige Xerogelkieselsäuren, Hydrogelkieselsäuren, Fällungskieselsäuren, Aluminiumoxid-trihydrat und feinteiliges alpha -Aluminiumoxid oder Mischungen dieser Putzkörper in Mengen von 15 bis 40 Gew.-% der Zahnpasta. Als Feuchthaltemittel kommen vorwiegend niedermolekulare Polyethylenglykole, Glycerin, Sorbit oder Mischungen dieser Produkte in Mengen bis zu 50 Gew.-% in Frage. Unter den bekannten Verdickungsmitteln sind die verdickenden, feinteiligen Gelkieselsäuren und Hydrokolloide, wie z. B. Carboxymethylcellulose, Hydroxyethylcellulose, Hydroxypropylguar, Hydroxyethylstärke, Polyvinylpyrrolidon, hochmolekulares Polyethylenglykol, Pflanzengummen wie Traganth, Agar-Agar, Carragheenmoos, Gummiarabicum, Xantham-Gum und Carboxyvinylpolymere (z. B. Carbopol^{®}-Typen) geeignet. Zusätzlich zu den Mischungen aus menthofuran und Mentholverbindungen können die Mund- und Zahnpflegemittel insbesondere oberflächenaktive Stoffe, bevorzugt anionische und nichtionische schaumstarke Tenside, wie die bereits oben genannten Stoffe, insbesondere aber Alkylethersulfat-Salze, Alkylpolyglucoside und deren Gemische.

Weitere übliche Zahnpastenzusätze sind:
- Konservierungsmittel und antimikrobielle Stoffe wie z. B. p- Hydroxybenzösäuremethyl-, -ethyl- oder -propylester, Natriumsorbat, Natriumbenzoat, Bromchlorophen, Phenylsalicylsäureester, Thymol und dergleichen;
- Antizahnsteinwirkstoffe, z. B. Organophosphate wie 1-Hydroxyethan- 1.1-diphosphonsäure, 1-Phosphonpropan-1,2,3-tricarbonsäure und andere, die z. B. aus US 3,488,419**,** DE 2224430 A1 und DE 2343196 A1 bekannt sind;
- andere karieshemmende Stoffe wie z. B. Natriumfluorid, Natriummonofluorphosphat, Zinnfluorid;
- Süssungsmittel, wie z. B. Saccharin-Natrium, Natrium-Cyclamat, Sucrose, Lactose, Maltose, Fructose oder Apartam^{®}, (L-Aspartyl- L-phenylalanin-methylester), Stivia-extrakte oder deren süßenden Bestandteile, insbesondere Ribeaudioside;
- Zusätzliche Aromen wie z. B. Eukalyptusöl, Anisöl, Fenchelöl, Kümmelöl, Methylacetat, Zimtaldehyd, Anethol, Vanillin, Thymol sowie Mischungen dieser und anderer natürlicher und synthetischer Aromen;
- Pigmente wie z. B. Titandioxid;
- Farbstoffe;
- Puffersubstanzen wie z. B. primäre, sekundäre oder tertiäre Alkaliphosphate oder Citronensäure/Natriumcitrat;
- wundheilende und entzündungshemmende Stoffe wie z. B. Allantoin, Harnstoff, Azulen, Kamillenwirkstoffe und Acetylsalicylsäurederivate.

Eine bevorzugte Ausführung der kosmetischen Zubereitungen sind Zahnpasten in Form einer wässrigen, pastösen Dispersion, enthaltend Poliermittel, Feuchthaltemittel, Viskositätsregulatoren und gegebenenfalls weitere übliche Komponenten, sowie die Mischung aus Menthofuran und Mentholverbindungen in Mengen von 0,5 bis 2 Gew.-% enthalten.

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

Als Konservierungsmittel eignen sich beispielsweise Phenoxyethanol, Formaldehydlösung, Parabene, Pentandiol oder Sorbinsäure sowie die unter der Bezeichnung Surfaeine^{®} bekannten Silberkomplexe und die in Anlage 6, Teil A und B der Kosmetikverordnung aufgeführten weiteren Stoffklassen.

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen Anethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### Hydrotrope

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

### Parfümöle, Aromen, Aromastoffe, Duftstoffe

Bevorzugt eingesetzte Duftstoffe bzw. Parfümöle, sind keinerlei Beschränkungen unterworfen. So können als Duftstoffe einzelne Riechstoffverbindungen, sowohl synthetische oder natürliche Verbindungen vom Typ der Ester, Ether, Aldehyde, Ketone, Alkohole, Kohlenwasserstoffe, Säuren, Kohlensäureester, aromatische Kohlenwasserstoffe, aliphatische Kohlenwasserstoffe, gesättigte und/oder ungesättigte Kohlenwasserstoffe und Mischungen daraus verwendet werden. Als Duftaldehyde oder Duftketone können dabei alle üblichen Duftaldehyde und Duftketone eingesetzt werden, die typischerweise zur Herbeiführung eines angenehmen Duftempfindens eingesetzt werden. Geeignete Duftaldehyde und Duftketone sind dem Fachmann allgemein bekannt. Die Duftketone können alle Ketone umfassen, die einen erwünschtem Duft oder ein Frischeempfinden verleihen können. Es können auch Gemische unterschiedlicher Ketone eingesetzt werden. Beispielsweise kann das Keton ausgewählt sein aus der Gruppe, bestehend aus Buccoxim, Iso jasmon, Methyl beta naphthyl keton, Moschus indanon, Tonalid/Moschus plus, Alpha-Damascon, Beta-Damascon, Delta-Damascon,Iso-Damascon, Damascenon, Damarose, Methyl-dihydrojasmonat, Menthon, Carvon, Campher, Fenchon, Alphalonen, Beta-Ionon, Dihydro-Beta-Ionon, Gamma-Methyl so genanntes Ionon, Fleuramon, Dihydrojasmon, Cis-Jasmon, Iso-E-Super, Methyl-cedrenyl-keton oder Methyl-cedrylon,Acetophenon, Methyl-acetophenon, Para-Methoxy-acetophenon, Methyl-beta-naphtyl-keton,Benzyl-aceton, Benzophenon, Para-Hydroxy-phenyl-butanon, Celery Keton oder Livescon,6-Isopropyldecahydro-2-naphton, Dimethyl-octenon, Freskomenth, 4-(I-Ethoxyvinyl)-3,3,5,5,-tetramethyl-cyclohexanon,Methyl-heptenon, 2-(2-(4-Methyl-3-cyclohexen-l-yl)propyl)-cyclopentanon, 1-(p-Menthen-6(2)-yl)-1-propanon,4-(4-Hydroxy-3-methoxyphenyl)-2-butanon, 2-Acetyl-3,3-dimethyl-norbornan, 6,7-Dihydro-1,1,2,3,3-pentamethyl-4(5H)-indanon,4-Damascol, Dulcinyl or Cassion, Gelson, Hexalon, Isocyclemon E, Methyl-cyclocitron, Methyl-Lavendel-keton, Orivon, Para-tert-butyl-cyclohexanon, Verdon, Delphon, Muscon, Neobutenon, Plicaton, Velouton, 2,4,4,7-Tetramethyl-oct-6-en-3-on, Tetrameran, Hedionund Gemischen davon. Bevorzugt können die Ketone ausgewählt sein aus AlphaDamascon, Delta Damascon, Iso Damascon, Carvon, Gamma-Methyl-ionon, Iso-E-Super,2,4,4,7-Tetramethyl-oct-6-en-3-on, Benzylaceton, Beta Damascon, Damascenon, Methyldihydrojasmonat, Methyl-cedrylon, Hedion und Gemischen davon.

Geeignete Duftaldehyde können beliebige Aldehyde sein, die entsprechend der Duftketone einen gewünschten Duft oder eine Frischeempfinden vermitteln. Es kann sich wiederum um einzelne Aldehyde oder Aldehydgemische handeln. Geeignete Aldehyde sind beispielsweise Melonal, Triplal, Ligustral, Adoxal, Anisaldehyd, Cymal,Ethylvanillin, Florhydral, Floralozon, Helional, Heliotropin, Hydroxycitronellal, Koavon, Laurinaldehyd, Canthoxal, Lyral, Lilial, Adoxal, Anisaldehyd, Cumal Methyl-nonyl-acetaldehyd, Citronellal, Citronellyloxyacetaldehyd, Cyclamenaldehyd, Bourgeonal, p,t-Bucinal, Phenylacetaldehyd, Undecylenaldehyd, Vanillin; 2,6,10-Trimethyl-9-undecenal, 3-Dodecen-I-al,alpha-n-Amylzimtaldehyd, 4-Methoxybenzaldehyd, Benzaldehyd, 3-(4-tert-Butylphenyl)-propanal,2-Methyl-3-(para-methoxyphenylpropanal), 2-Methyl-4-(2,6,6-trimethyl-2(1)-cyclohexen-1-yl)butanal,3-Phenyl-2-propenal, cis-/trans-3,7-Dimethyl-2,6-octadien-I-al, 3,7-Dimethyl-6-octen-I-al,[(3,7-Dimethyl-6-octenyl)oxy]acetaldehyd, 4-Isopropylbenzyaldehyd, 1,2,3,4,5,6,7,8-octahydro-8,8-Dimethyl-2-naphthaldehyd,2,4-Dimethyl-3-cyclohexen-1-carboxyaldehyd, 2-Methyl-3-(isopropylphenyl)propanal, Decyl aldehyd, 2,6-Dimethyl-5-heptenal; 4-(tricyclo[5.2. 1.0 (2,6)]-decylidene-8)-butanal;Octahydro-4,7-methano-IH-indenecarboxaldehyd; 3-Ethoxy-4-hydroxybenzaldehyd, para-Ethyl-alpha,alpha-dimethylhydrozimtaldehyd, alpha-Methyl-3,4-(methylenedioxy)-hydrozimtaldehyd,3,4-Methylenedioxybenzaldehyd, alpha-n-Hexylzimtaldehyd, m-Cymene-7-carboxaldehyd,alpha-Methylphenylacetaldehyd, 7-Hydroxy-3,7-dimethyl octanal, Undecenal, 2,4,6-Trimethyl-3-cyclohexene-I-carboxaldehyd,4-(3)(4-Methyl-3-pentenyl)-3-cyclohexen-carboxaldehyd, 1-Dodecanal, 2,4-Dimethylcyclohexene-3-carboxaldehyd,4-(4-Hydroxy-4-methyl pentyl)-3-cylohexene-I-carboxaldehyd, 7-Methoxy-3,7-dimethyloctan-1-al,2-Methyl undecanal, 2-Methyl decanal, 1-nonanal, 1-Octanal, 2,6,10-Trimethyl-5,9-undecadienal,2-Methyl-3-(4-tertbutyl)propanal, 3-(4-Ethylphenyl)-2,2-Dimethylpropanal, 3-(4-Methoxyphenyl)-2-methylpropanal,Methylnonylacetaldehyd, 2-Phenylpropan-1-al, 3-Phenylprop-2-en-1-al, 3-Phenyl-2-pentylprop-2-en-1-al,3-Phenyl-2-hexylprop-2-enal, 3-(4-Isopropylphenyl)-2-methylpropan-1-al, 3-(4-Ethylphenyl)-2,2-dimethylpropan-1-al,3-(4-tert-Butylphenyl)-2-methyl-propanal, 3-(3,4-Methylendioxyphenyl)-2-methylpropan-1-al,3-(4-Ethylphenyl)-2,2-dimethylpropanal, 3-(3-Isopropylphenyl)-butan-1-al, 2,6-Dimethylhept-5-en-1-al,Dihydrozimtaldehyd, 1-methyl-4-(4-methyl-3-pentenyl)-3-cyclohexene-1-carboxaldehyd,5 oder 6 Methoxyhexahydro-4,7-methanoindan-1 oder 2-carboxyaldehyd, 3,7-Dimethyloctan-1-al,l-Undecana1,10-Undecen-1-al, 4-Hydroxy-3-methoxybenzaldehyd, 1-Methyl-3-(4-methylpentyl)-3-cyclohexene-carboxyaldehyd,7-Hydroxy-3,7-dimethyl-octanal; trans-4-decenal, 2,6-Nonadienal, para-Tolylacetaldehyd;4-Methylphenylacetaldehyd, 2-Methyl-4-(2,6,6-trimethyl-1-cyclohexen-1-yl)-2-butenal,ortho-Methoxyzimtaldehyd, 3,5,6-Trimethyl-3-cyclohexenecarboxaldehyd, 3,7-Dimethyl-2-methylene-6-octenal,Phenoxyacetaldehyd; 5,9-Dimethyl-4,8-decadienal, Peony aldehyd (6,10-dimethyl-3-oxa- 5,9-undecadien-1-al),Hexahydro-4,7-methanoindan-1-carboxaldehyd, Octanal, 2-Methyl octanal, alpha-Methyl-4-(I-methylethyl)benzeneacetaldehyd,6,6-Dimethyl-2-norpinene-2-propionaldehyd, para Methyl phenoxy acetaldehyd, 2-methyl-3-phenyl-2-propen-1-al,3,5,5-Trimethylhexanal, Hexahydro-8,8-dimethyl-2-naphthaldehyd, 3-Propyl-bicyclo[2.2.1]-hept-5-ene-2-carbaldehyd,9-Decenal, 3-Methyl-5-phenyl-1-pentanal, Methyl-nonyl acetaldehyd, 1-p-Menthene-q-carboxaldehyd,Citral oder Gemische davon, Lilial citral, 1-Decanal, n-Undecanal, n-Dodecanal, Florhydral, 2,4-Dimethyl-3-cyclohexen-1-carboxaldehyd 4-Methoxybenzaldehyde, 3-Methoxy-4-hydroxybenzaldehyde,3-Ethoxy-4-hydroxybenzaldehyde, 3,4-Methylendioxybenzaldehyd und 3,4-Dimethoxybenzaldehydund Gemische davon. Wie vorstehend beispielhaft ausgeführt, können die Duftaldehyde und Duftketone eine aliphatische, cycloaliphatische, aromatische, ethylenisch ungesättigte Struktur oder eine Kombination dieser Strukturen aufweisen. Es können fernerweitere Heteroatome oder polycyclische Strukturen vorliegen. Die Strukturen können geeignete Substituenten wie Hydroxyl- oder Aminogruppen aufweisen. Für weitere geeignete Duftstoffe, ausgewählt aus Aldehyden und Ketonen, wird auf Steffen Arctander "Published 1960 and 1969 respectively, Reprinted2000 ISBN: Aroma Chemicals Vol. 1: 0-931710-37-5, Aroma Chemicals Vol. 2: 0-931710-38-3", verwiesen.

Geeignete Riechstoffverbindungen vom Typ der Ester sind beispielsweise Benzylacetat, Phenoxyethylisobutyrat, p-tert.-Butylcyclohexylacetat, Linalylacetat, Dimethylbenzylcarbinylacetat (DMBCA), Phenylethylacetat, Benzylacetat, Ethylmethylphenylglycinat, Allylcyclohexylpropionat, Styrallylpropionat, Benzylsalicylat, Cyclohexylsalicylat, Floramat, Melusat und Jasmacyclat. Riechstoffverbindungen vom Typ der Kohlenwasserstoffen sind z.B. Terpene wie Limonen und Pinen. Geeignete Duftstoffe vom Typ Ether sind beispielsweise Benzylethylether und Ambroxan. Geeignete Duftstoffalkohole sind beispielsweise 10-Undecen-l-ol, 2,6-Dimethylheptan-2-ol, 2-Methylbutanol, 2-Methylpentanol,2-Phenoxyethanol, 2-Phenylpropanol, 2-tert-Butycyclohexanol, 3,5,5-Trimethylcyclohexanol,3-Hexanol, 3-Methyl-5-phenylpentanol, 3-Octanol, 1-Octen-3-ol, 3-Phenylpropanol,4-Heptenol, 4-Isopropylcyclohexanol, 4-tert-Butycyclohexanol, 6,8-Dimethyl-2-nonanol,6-Nonen-l-ol, 9-Decen-1-ol, alpha-Methylbenzylalkohol, alpha-Terpineol, Amylsalicylat,Benzylalkohol, Benzylsalicylat, beta-Terpineol, Butylsalicylat,Citronellol, Cyclohexylsalicylat, Decanol, Dihydromyrcenol, Dimethylbenzylcarbinol,Dimethylheptanol, Dimethyloctanol, Ethylsalicylat, Ethylvanilin, Anethol, Eugenol,Geraniol, Heptanol, Hexylsalicylat, Isoborneol, Isoeugenol, Isopulegol, Linalool,Menthol, Myrtenol, n-Hexanol, Nerol, Nonanol, Octanol, para-Menthan-7-ol, Phenylethylalkohol,Phenol, Phenylsalicylat, Tetrahydrogeraniol, Tetrahydrolinalool, Thymol, trans-2-cis-6-Nonadienol,trans-2-Nonen-1-ol, trans-2-Octenol, Undecanol, Vanillin, Zimtalkohol, wobei, wenn mehrere Duftstoffalkohole vorhanden sind, diese unabhängig voneinander ausgewählt sein können.

Duftstoffe bzw. Parfümöle können auch natürliche Riechstoffgemische sein, wie sie aus pflanzlichen Quellen zugänglich sind, z.B. Pine-, Citrus-, Jasmin-, Patchouly-, Rosen- oder Ylang-Ylang-öl. Ebenfallsgeeignet sind Muskateller-Salbeiöl, Kamillenöl, Nelkenöl, Melissenöl,Minzöl, Zimtblaetteröl, Lindenbluetenöl, Wacholderbeeröl, Vetiveröl, Olibanumöl, Galbanumöl und Labdanumöl sowie Orangenblütenöl, Neroliöl, Orangenschalenöl und Sandelholzöl. Ätherischen Öle wie Angelikawurzelöl, Anisöl, Arnikablütenöl, Basilikumöl, Bayöl, Champacablütenöl, Edeltannenöl, Edeltannenzapfenöl, Elemiöl, Eukalyptusöl, Fenchelöl, Fichtennadelöl, Galbanumöl, Geraniumöl, Gingergrasöl, Guajakholzöl, Gurjunbalsamöl, Helichrysumöl, Ho-öl, Ingweröl, Irisöl, Kajeputöl, Kalmusöl, Kamillenöl, Kampferöl, Kanagaöl, Kardamomenöl, Kassiaöl, Kiefernnadelöl, Kopaivabalsamöl, Korianderöl, Krauseminzeöl, Kümmelöl, Kuminöl, Lavendelöl, Lemongrasöl, Limette-öl, Mandarinenoel, Melissenöl, Moschuskerneröl, Myrrhenöl, Nelkenöl, Neroliöl, Niaouliöl, Olibanumöl, Origanumöl, Palmarosaöl, Patchuliöl,Perubalsamöl, Petitgrainöl, Pfefferöl, Pfefferminzöl, Pimentöl, Pine-öl, Rosenöl, Rosmarinöl, Sandelholzöl, Sellerieöl, Spiköl, Sternanisöl, Terpentinöl, Thujaöl, Thymianöl, Verbenaöl, Vetiveröl, Wacholderbeeröl, Wermutöl, Wintergrünöl, Ylang-Ylang-öl, Ysop-öl, Zimtöl, Zimtblätteröl, Zitronellöl, Zitronenöl sowie Zypressenöl.

Ebenfalls als Duftstoff geeignet sind sogenannte Duftstoffvorläufer (Pro-Drug). Bei dieser Klasse von Verbindungen handelt es sich um Verbindungen, welche durch das Aufbrechen einer chemischen Bindung, beispielsweise durch Hydrolyse, ein erwünschtes Geruchs- und/oder Duftstoffmolekül freisetzt. Typischerweise wird zur Bildung eines Duftstoffvorläufers ein gewünschtes Duftstoffrohmaterial chemisch mit einem Träger, vorzugsweise einem geringfügig flüchtigen oder mäßig flüchtigen Träger, verbunden. Die Kombination führt zu einem weniger flüchtigen und stärker hydrophoben Duftstoffvorläufer mit verbesserter Anlagerung auf Stoffen. Der Duftstoff wird danach durch Aufbrechen der Bindung zwischen dem Duftstoffrohmaterial und dem Träger freigesetzt, beispielsweise durch eine Veränderung des pH-Werts (z. B. durch Transpiration beim Tragen), Luftfeuchtigkeit, Wärme und/oder Sonnenlicht während der Lagerung oder des Trocknens auf der Wäscheleine.

Das Duftstoffrohmaterial für Verwendung in Duftstoffvorläufern sind typischerweise gesättigte oder ungesättigte, flüchtige Verbindungen, die einen Alkohol, einen Aldehyd und/oder eine Ketongruppe enthalten. Zu den hierin nützlichen Duftstoffrohmaterialien gehören jegliche wohlriechenden Substanzen oder Mischungen von Substanzen, die bereits oben beschrieben wurden.

Besondere vorteilhafte, einsetzbare Duftstoffvorläufer gehorchen der Formel (III)

R-C(OR¹)(OR²)-OR³ (III)

worin R Wasserstoff, lineares C₁-C₈-Alkyl, verzweigtes C₃-C₂₀-Alkyl, cyclisches C₃-C₂₀-Alkyl, verzweigtes cyclisches C₆-C₂₀-Alkyl, lineares C₆-C₂₀-Alkenyl, verzweigtes C₆-C₂₀-Alkenyl, cyclisches C₆-C₂₀-Alkenyl, verzweigtes cyclisches C₆-C₂₀-Alkenyl, substituiertes oder unsubstituiertes C₆-C₂₀-Aryl und Mischungen hiervon bedeutet; R¹, R² und R³ unabhängig lineares, verzweigtes oder substituiertes C₁-C₂₀-Alkyl; lineares, verzweigtes oder substituiertes C₂-C₂₀-Alkenyl; substituiertes oder unsubstituiertes, cyclisches C₃-C₂₀-Alkyl; substituiertes oder substituiertes C₆-C₂₀-Aryl, substituiertes oder unsubstituiertes C₂-C₄₀-Alkylenoxy; substituiertes oder unsubstituiertes C₃-C₄₀-Alkylenoxyalkyl; substituiertes oder unsubstituiertes C₆-C₄₀-Alkylenaryl; substituiertes oder unsubstituiertes C₆-C₃₂-Aryloxy; substituiertes oder unsubstituiertes C₆-C₄₀-Alkylenoxyaryl; C₆-C₄₀-Oxyalkylenaryl und Mischungen hiervon bedeuten. Der Einsatz solcher Substanzen, insbesondere in (vorzugsweise wasserunlöslichen) Mikrokapseln, entspricht einer bevorzugten Ausführungsform der Erfindung.

Weitere besonders vorteilhafte, einsetzbare Duftstoffvorläufer, sind Acetale oder Ketale, vorzugsweise gehorchend der Formel (IV)

R-C(R¹)(OR³)-OR2 (IV)

worin R lineares C₁-C₂₀-Alkyl, verzweigtes C₃-C₂₀-Alcyl, cyclisches C₆-C₂₀-Alkyl, verzweigtes cyclisches C₆-C₂₀-Alkyl, lineares C₂-C₂₀-Alkenyl, verzweigtes C₃-C₂₀-Alkenyl, cyclisches C₆-C₂₀-Alkenyl, verzweigtes cyclisches C₆-C₂₀-Alkenyl, substituiertes oder unsubstituiertes C₆-C₂₀-Aryl und Mischungen hiervon ist; R¹ Wasserstoff oder R ist; R² und R³ jeweils unabhängig voneinander gewählt sind aus der Gruppe, bestehend aus lineares C₁-C₂₀-Alkyl, verzweigtes C₃-C₂₀-Alkyl, cyclisches C₃-C₂₀-Alkyl, verzweigtes cyclisches C₆-C₂₀-Alkyl, lineares C₆-C₂₀-Alkenyl, verzweigtes C₆-C₂₀-Alkenyl, cyclisches C₆-C₂₀-Alkenyl, verzweigtes cyclisches C₆-C₂₀-Alkenyl, C₆-C₂₀-Aryl, substituiertes C₇-C₂₀-Aryl und Mischungen hiervon. Der Einsatz solcher Substanzen, insbesondere in (vorzugsweise wasserunlöslichen) Mikrokapseln, entspricht einer bevorzugten Ausführungsform der Erfindung.

Weitere besonders vorteilhafte, einsetzbare Duftstoffvorläufer gehorchen der Formel (V)

R⁴O-C(OR¹)(OR³)-OR² (V)

worin R¹, R², R³ und R⁴ unabhängig voneinander lineares, verzweigtes oder substituiertes C₁-C₂₀-Alkyl; lineares, verzweigtes oder subsituiertes C₂-C₂₀-Alkenyl; substituiertes oder unsubstituiertes, cyclisches C₅-C₂₀-Alkyl; substituiertes oder unsubstituiertes C₆-C₂₀-Aryl, substituiertes oder unsubstituiertes C₂-C₄₀-Alkylenoxy; substituiertes oder unsubstituiertes C₃-C₄₀-Alkylenoxyalkyl; substituiertes oder unsubstituiertes C₆-C₄₀-Alkylenaryl; substituiertes oder unsubstituiertes C₆-C₃₂-Aryloxy; substituiertes oder unsubstituiertes C₆-C₄₀-Alkylenoxyaryl; C₆-C₄₀-Oxyalkylenaryl; und Mischungen hiervon sind. Der Einsatz solcher Substanzen, insbesondere in (vorzugsweise wasserunlöslichen) Mikrokapseln, entspricht einer bevorzugten Ausführungsform der Erfindung.

Besonders bevorzugt ist es, wenn die eingesetzten Riechstoffe Kieselsäureester-Mischungen umfassen. Kieselsäureester werden beispielsweise durch die Formel (V)

R-(-O-Si (OR)₂-)ₙ-OR (V)

beschrieben, wobei R unabhängig voneinander ausgewählt ist aus der Gruppe, die H, die geradkettigen oder verzweigten, gesättigten oder ungesättigten, substituierten oder unsubstituierten C₁-C₆-Kohlenwasserstoffreste und die Duftstoffalkoholreste und/oder Biozidalkoholreste enthält, und m Werte aus dem Bereich 1 bis 20 und n Werte aus dem Bereich 2 bis 100 annimmt. Vorzugsweise enthalten die Kieselsäureester der Formeln zumindest einen Duftstoffalkoholrest und/oder Biozidalkoholrest.

Die Kieselsäureester-Mischungen können verkapselt, aber auch unverkapselt zum Einsatz kommen. Die Anwesenheit von Kieselsäureester-Mischungen führt oftmals dazu, dass der erzielbare Dufteindruck, sowohl was Gefallen als auch Intensität anbetrifft, noch weiter verbessert werden kann. Der Dufteindruck ist nicht nur qualitativ, d. h. das Gefallen anbetreffend, besser, sondern halt auch länger an.

Die Kieselsäureester-Mischungen können auch in den Mikrokapseln enthalten sein. Wenn die Kieselsäureester-Mischungen in den Mikrokapseln vorzugsweise mindestens 2 Gew.- % der gesamten verkapselten Riechstoffmenge ausmachen, Gew.- % bezogen auf die Menge der verkapselten Riechstoffe, so liegt eine bevorzugte Ausführungsform der Erfindung vor, welche eine weitere Verbesserung des angestrebten Wohlgeruchseffektes nach dem Trocknen bewirkt.

Besonders geeignete Duftstoffvorläufer sind Reaktionsprodukte von Verbindungen, die mindestens eine primäre und/oder sekundäre Amingruppe umfassen, beispielsweise einem aminofunktionellen Polymer, insbesondere einem aminofunktionellen Silikon, und einem Duftstoffbestandteil, der aus Keton, Aldehyd und Mischungen davon ausgewählt ist. Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

Aromastoffe umfassen beispielsweise: Acetophenon, Allylcapronat, alpha-Ionon, beta-Ionon, Anisaldehyd, Anisylacetat, Anisylformiat, Benzaldehyd, Benzothiazol, Benzylacetat, Benzylalkohol, Benzylbenzoat, beta-Ionon, Butylbutyrat, Butylcapronat, Butylidenphthalid, Carvon, Camphen, Caryophyllen, Cineol, Cinnamylacetat, Citral, Citronellol, Citronellal, Citronellylacetat, Cyclohexylacetat, Cymol, Damascon, Decalacton, Dihydrocumarin, Dimethylanthranilat, Dimethylanthranilat, Dodecalacton, Ethoxyethylacetat, Ethylbuttersäure, Ethylbutyrat, Ethylcaprinat, Ethylcapronat, Ethylcrotonat, Ethylfuraneol, Ethylguajakol, Ethylisobutyrat, Ethylisovalerianat, Ethyllactat, Ethylmethylbutyrat, Ethylpropionat, Eucalyptol, Eugenol, Ethylheptylat, 4-(p-Hydroxyphenyl)-2-butanon, gamma-Decalacton, Geraniol, Geranylacetat, Geranylacetat, Grapefruitaldehyd, Methyldihydrojasmonat (z.B. Hedion^{®}), Heliotropin, 2-Heptanon, 3-Heptanon, 4-Heptanon, trans-2-Heptenal, cis-4-Heptenal, trans-2-Hexenal, cis-3-Hexenol, trans-2-Hexensäure, trans-3-Hexensäure, cis-2-Hexenylacetat, cis-3-Hexenylacetat, cis-3-Hexenylcapronat, trans-2-Hexenylcapronat, cis-3-Hexenylformiat, cis-2-Hexylacetat, cis-3-Hexylacetat, trans-2-Hexylacetat, cis-3-Hexylformiat, para-Hydroxybenzylaceton, Isoamylalkohol, Isoamylisovalerianat, Isobutylbutyrat, Isobutyraldehyd, Isoeugenolmethylether, Isopropylmethylthiazol, Laurinsäure, Leavulinsäure, Linalool, Linalooloxid, Linalylacetat, Menthol, Menthofuran, Methylanthranilat, Methylbutanol, Methylbuttersäure, 2-Methylbutylacetat, Methylcapronat, Methylcinnamat, 5-Methylfurfural, 3,2,2-Methylcyclopentenolon, 6,5,2-Methylheptenon, Methyldihydrojasmonat, Methyljasmonat, 2-Methylmethylbutyrat, 2-Methyl-2-Pentenolsäure, Methylthiobutyrat, 3,1-Methylthiohexanol, 3-Methylthiohexylacetat, Nerol, Nerylacetat, trans,trans-2,4-Nonadienal, 2,4-Nonadienol, 2,6-Nonadienol, 2,4-Nonadienol, Nootkaton, delta Octalacton, gamma Octalacton, 2-Octanol, 3-Octanol, 1,3-Octenol, 1-Octylacetat, 3-Octylacetat, Palmitinsäure, Paraldehyd, Phellandren, Pentandion, Phenylethylacetat, Phenylethylalkohol, Phenylethylalkohol, Phenylethylisovalerianat, Piperonal, Propionaldehyd, Propylbutyrat, Pulegon, Pulegol, Sinensal, Sulfurol, Terpinen, Terpineol, Terpinolen, 8,3-Thiomenthanon, 4,4,2-Thiomethylpentanon, Thymol, delta-Undecalacton, gamma-Undecalacton, Valencen, Valeriansäure, Vanillin, Acetoin, Ethylvanillin, Ethylvanillinisobutyrat (= 3-Ethoxy-4-isobutyryloxybenzaldehyd), 2,5-Dimethyl-4-hydroxy-3(2H)-furanon und dessen Abkömmlinge (dabei vorzugsweise Homofuraneol (= 2-Ethyl-4-hydroxy-5-methyl-3(2H)-furanon), Homofuronol (= 2-Ethyl-5-methyl-4-hydroxy-3(2H)-furanon und 5-Ethyl-2-methyl-4-hydroxy-3(2H)-furanon), Maltol und Maltol-Abkömmlinge (dabei vorzugsweise Ethylmaltol), Cumarin und Cumarin-Abkömmlinge, gamma-Lactone (dabei vorzugsweise gamma-Undecalacton, gamma-Nonalacton, gamma-Decalacton), delta-Lactone (dabei vorzugsweise 4-Methyldeltadecalacton, Massoilacton, Deltadecalacton, Tuberolacton), Methylsorbat, Divanillin, 4-Hydroxy-2(oder 5)-ethyl-5(oder 2)-methyl-3(2H)furanon, 2-Hydroxy-3-methyl-2-cyclopentenon, 3-Hydroxy-4,5-dimethyl-2(5H)-furanon, Essigsäureisoamylester, Buttersäureethylester, Buttersäure-n-butylester, Buttersäureisoamylester, 3-Methyl-buttersäureethylester, n-Hexansäureethylester, n-Hexansäureallylester, n-Hexansäure-n-butylester, n-Octansäureethylester, Ethyl-3-methyl-3-phenylglycidat, Ethyl-2-trans-4-cis-decadienoat, 4-(p-Hydroxyphenyl)-2-butanon, 1,1-Dimethoxy-2,2,5-trimethyl-4-hexan, 2,6-Dimethyl-5-hepten-1-al und Phenylacetaldehyd, 2-Methyl-3-(methylthio)furan, 2-Methyl-3-furanthiol, bis(2-Methyl-3-furyl)disulfid, Furfurylmercaptan, Methional, 2-Acetyl-2-thiazolin, 3-Mercapto-2-pentanon, 2,5-Dimethyl-3-furanthiol, 2,4,5-Trimethylthiazol, 2-Acetylthiazol, 2,4-Dimethyl-5-ethylthiazol, 2-Acetyl-1-pyrrolin, 2-Methyl-3-ethylpyrazin, 2-Ethyl-3,5-dimethylpyrazin, 2-Ethyl-3,6-dimethylpyrazin, 2,3-Diethyl-5-methylpyrazin, 3-Isopropyl-2-methoxypyrazin, 3-Isobutyl-2-methoxypyrazin, 2-Acetylpyrazin, 2-Pentylpyridin, (E,E)-2,4-Decadienal, (E,E)-2,4-Nonadienal, (E)-2-Octenal, (E)-2-Nonenal, 2-Undecenal, 12-Methyltridecanal, 1-Penten-3-on, 4-Hydroxy-2,5-dimethyl-3(2H)-furanon, Guajakol, 3-Hydroxy-4,5-dimethyl-2(5H)-furanon, 3-Hydroxy-4-methyl-5-ethyl-2(5H)-furanon, Zimtaldehyd, Zimtalkohol, Methylsalicylat, Isopulegol sowie (hier nicht explizit genannte) Stereoisomere, Enantiomere, Stellungsisomere, Diastereomere, cis/trans-Isomere bzw. Epimere dieser Substanzen.

Bevorzugt werden Mischungen verschiedener Duftstoffe (aus den verschiedenen oben genannten Duftstoffklassen) verwendet, die gemeinsam eine ansprechende Duftnote erzeugen. In diesem Fall ist die Gesamtmenge des mindestens einen Duftstoffs die Menge aller Duftstoffe in der Mischung zusammen bezogen auf die Gesamtmenge des Mittels.

### Farbstoffe

Als Farbstoffe können die für kosmetische Zwecke geeigneten und zugelassenen Substanzen verwendet werden, wie sie beispielsweise in der Publikation "Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbtoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

Ebenfalls geeignet sind die bekannten zugelassenen Lebensmittelfarbstoffe:

| | | |
|---|---|---|
| Allurarot AC | E 129 | rot |
| Aluminium | E 173 | silbrig-grau |
| Amaranth | E 123 | rot |
| Anthocyane | E 163 | violett, blau |
| Azorubin | E 122 | rot |
| Betanin | E 162 | rot |
| Braun FK | E 154 | gelb-braun |
| Braun HT | E 155 | rot-braun |
| Brillantblau FCF | E 133 | blau |
| Brillantschwarz BN | E 151 | violett, braun, schwarz |
| Calciumcarbonat | E 170 | |
| Canthaxanthin | E 161 g | |
| Carotin | E 160 a | |
| * Annatto (Norbixin) | E 160 b | |
| * Capsanthin | E 160 c | |
| * Lycopin | E 160 d | |
| * 8'-Apo-β-caroten-8'-al | E 160 e | |
| * Ethyl-8'-apo-β-caroten-8'-oat | E 160 f | |
| Chinolingelb | E 104 | |
| Chlorophyll | E 140 | grün |
| Cochenillerot A | E 124 | |
| Curcumin | E 100 | |
| Eisenoxid | E 172 | |
| Erythrosin | E 127 | |
| Gelborange S | E 110 | |
| Gold | E 175 | |
| Grün S | E 142 | |
| Indigotin | E 132 | |
| Koschenille | E 120 | |
| Kupferhaltige Komplexe der | | |
| Chlorophylle und Chlorophylline | E 141 | |
| Lactoflavin | E 101 | |
| Litholrubin BK | E 180 | |
| Lutein | E 161 b | |
| Patentblau V | E 131 | |
| Pflanzenkohle | E 153 | |
| Riboflavin (Vitamin B2) | E 101 | |
| * Riboflavin-5-phosphat | E 101 a | |
| Saflor kirschrot bis braungelb | | |
| Silber | E 174 | |
| Tartrazin | E 102 | Zitronengelb |
| Titandioxid | E 171 | |
| Zuckerkulör | E 150 a | |
| * Sulfitlaugen-Zuckerkulör | E 150 b | |
| * Ammoniak-Zuckerkulör | E 150 c | |
| * Ammonsulfit-Zuckerkulör | E 150 d | |
| Zeaxanthin | E 161 h | |

### Verwendete Abkürzungen

Dipropylenglycol (DPG), Diethylphthalat (DEP), Triethylcitrat (TEC), Isopropylmyristat (IPM); nat. = natürlich;
Für Erläuterungen der Produktnamen der Riechstoffe siehe z.B. S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

### BEISPIELE

### Allgemeine Herstellvorschrift einer Verbindung der Formel (I)

### Beispiel 1

### Herstellungsbeispiel: l-Cyclopent-2-en-1-yl propan-2-one

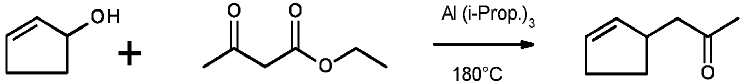

In einem 100ml-Dreihalskolben mit Magnetrührer, Kontaktthermometer, Tropftrichter, 5cm-Vigreux-Kolonne und Destbrücke werden 69mg (0,34mmol) Aluminiumtriisopropylat und 4,6g (0,035mol) Acetessigester vorgelegt und auf 180°C aufgeheizt. Bei dieser Temperatur wird eine Lösung aus 12,6g (0,15mol) 2-Cylopentanol und 21g (0,16mol) Acetessigester in 2h, unter stetiger Destillatabnahme und Kohlendioxidentwicklung, zugetropft. Es wird noch 2h bei 180°C nachgerührt. Der Rückstand (13g) wird an der Kugelrohr destilliert (Siedebereich: 110°C/80-40mbar). Man erhält 8,1g Destillat (GC: 84% Produkt). 2,7g des Destillats werden an 120g Kieselgel 60 mit Hexan/Essigester präparativ gesäult (Büchi PrepChrom C-700, Säule: 120g (SepaCore)). Ausbeute: 1,5g (GC: 99% Produkt).

### Beispiel 2

### Liste von Verbindungen der allgeminen Formel (I) mit Geruchsbeschreibungen und MS-Daten

### Methyl 2-cyclopent-2-en-1-ylacetate (Verbindung II)

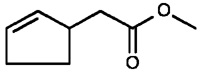

GCMS: m/z (%) = 140(11), 109(15), 108(20), 80(33), 79(31), 74(43), 67(100), 66(25), 43(16), 41(23), 39(20)
Geruchsprofil: fruchtig in Richtung Erdbeere

### Ethyl 2-cyclopent-2-en-1-ylacetate (Verbindung III)

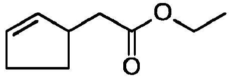

GCMS: m/z (%) = 154(31), 109(25), 108(27), 88(39), 83(36), 80(47), 79(64), 67(100), 66(28), 41(23)
Geruchsprofil: süß, fruchtig, grün, in Richtung Birne Ananas Aprikose und Kiwi

### Propyl 2-cyclopent-2-en-1-ylacetate (Verbindung IV)

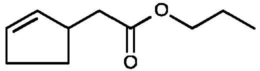

GCMS: m/z (%) = 168(6), 109(30), 108(27), 107(20), 83(26), 80(32), 79(44), 67(100), 66(31), 61(22), 41(28)
Geruchsprofil: süß, fruchtig, grün, fettig, in Richtung Ananas

### Isopropyl 2-cyclopent-2-en-1-ylacetate (Verbindung V)

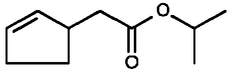

GCMS: m/z (%) = 168(1), 126(16), 125(15), 109(21), 108(29), 80(15), 79(26), 67(100), 66(34), 43(17), 41(17)
Geruchsprofil: süß, fruchtig, Bergamott und Veilchen

### Allyl 2-cyclopent-2-en-1-ylacetate (Verbindung VI)

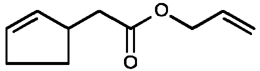

GCMS: m/z (%) = 125(56), 107(42), 83(62), 80(18), 79(100), 77(12), 67(82), 66(14), 41(56), 39(29)
Geruch: süß, fruchtig, grün, in Richtung Ananas

### Isopentyl 2-cyclopent-2-en-1-ylacetate (Verbindung VII)

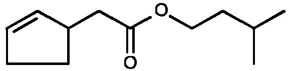

GCMS: m/z (%) = 196(4), 80(23), 79(33), 71(51), 70(27), 67(81), 66(23), 43(100), 41(52), 39(25), 27(22)
Geruchsprofil: süß, fruchtig, grün, in Richtung Apfel und Birne

### Cyclopentyl 2-cyclopent-2-en-1-ylacetate (Verbindung VIII)

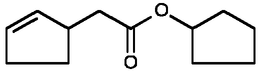

GCMS: m/z (%) = 126(18), 109(21), 108(37), 80(13), 79(13), 69(9), 68(9), 67(100), 66(25), 41(22)
Geruchsprofil: süß, fruchtig, in Richtung Ananas

### [(E)-But-2-enyl] 2-cyclopent-2-en-1-ylacetate (Verbindung IX)

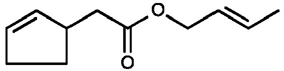

GCMS: m/z (%) = 125(65), 108(17), 107(41), 83(51), 79(76), 67(100), 66(26), 55(49), 41(22), 39(22)
Geruchsprofil: süß, pilzig, fruchtig, in Richtung Ananas

### Cyclopropylmethyl 2-cyclopent-2-en-1-ylacetate (Verbindung X)

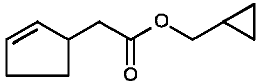

GCMS: m/z (%) = 125(33), 107(38), 83(35), 80(19), 79(56), 67(80), 55(100), 41(17), 39(17), 29(21)
Geruchsprofil: süß, pilzig, fruchtig, in Richtung Ananas

### 1,2-Dimethylpropyl 2-cyclopent-2-en-1-ylacetate (Verbindung XI)

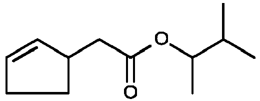

GCMS: m/z (%) = 126(14), 109(57), 108(25), 79(13), 71(43), 70(24), 67(100), 66(19), 43(42), 41(15)
Geruchsprofil: süß, grün, fruchtig, in Richtung Ananas

### Ethyl 2-(cyclopenten-1-yl) acetate (Verbindung XII)

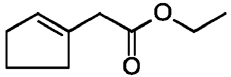

GCMS: m/z (%) = 154(36), 125(6), 108(39), 88(9), 83(36), 81(100), 67(45), 53(21), 41(22)
Geruchsprofil: süß, frisch, fruchtig, in Richtung Apfel

### Methyl 2-cyclopentylideneacetate(Verbindung XIII)

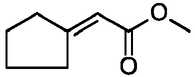

GCMS: m/z (%) = 140(100), 125(7), 109(66), 81(42), 74(26), 67(31), 53(25), 41(28)
Geruchsprofil: süß, grün, fruchtig, in Richtung Banane, floral nach Ylang und honigartig

### Methyl 2-cyclopentylacetate (Verbindung XIV)

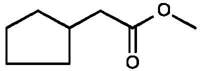

GCMS: m/z (%) = 142(1), 111(11), 99(10), 83(11), 75(28), 74(100), 67(11), 59(10), 55(22), 43(23), 41(20)
Geruchsprofil: süß, fruchtig, in Richtung Valerianat

### Propyl 2-cyclopentylacetate (Verbindung XV)

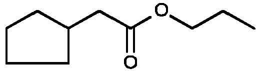

GCMS: m/z (%) = 129(43), 111(56), 102(17), 83(48), 67(21), 61(100), 60(48), 55(34), 43(24), 41(35)
Geruchsprofil: süß, fruchtig, in Richtung Banane

### Allyl 2-cyclopentylacetate (Verbindung XVI)

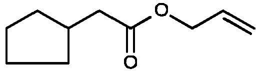

GCMS: m/z (%) = 168(2), 111(50), 100(36), 83(100), 82(17), 67(45), 55(76), 54(19), 43(24), 41(87), 39(24)
Geruchsprofil: süß, grün, fruchtig, in Richtung Ananas

### Isopropyl 2-cyclopentylacetate (Verbindung XVII)

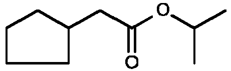

GCMS: m/z (%) = 129(34), 111(63), 102(48), 83(67), 68(32), 61(62), 60(100), 55(43), 43(69), 41(48)
Geruch: süß, fruchtig, in Richtung Banane, floral rosig

### [(E)-But-2-enyl] 2-cyclopentylacetate (Verbindung XVIII)

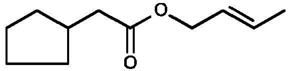

GCMS: m/z (%) = 182(4), 111(50), 109(12), 83(77), 67(23), 55(100), 54(14), 41(19), 39(13), 29(16), 27(10)
Geruchsprofil: süß, fruchtig, in Richtung Ananas, agrumenartig

### 3-Methylbut-2-enyl 2-cyclopent-2-en-1-ylacetate (Verbindung XIX)

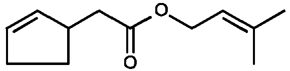

GCMS: m/z (%) = 126(23), 125(31), 108(29), 83(22), 79(32), 69(79), 68(39), 67(100), 66(22), 41(62)
Geruchsprofil: süß, fruchtig

### Ethyl 2-cyclopentylacetate (Verbindung XX)

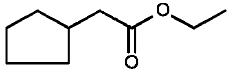

GCMS: m/z (%) = 156(1), 111(26), 89(23), 88(100), 83(30), 70(33), 61(27), 60(28), 55(29), 41(33), 29(22)
Geruchsprofil: süß, fruchtig, in Richtung Apfel und Erdbeere

### 1-Cyclopent-2-en-1-ylpropan-2-one (Verbindung XXII)

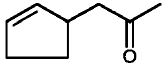

GCMS: m/z (%) = 124(34), 109(27), 81(20), 79(21), 67(72), 66(100), 65(11), 43(68), 41(18), 39(18)
Geruchsprofil: grün, fruchtig, nussig

### 1-Cyclopent-2-en-1-ylhexan-2-one (Verbindung XXII)

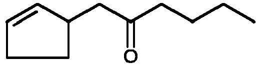

GCMS: m/z (%) = 166(9), 109(100), 85(15), 81(17), 79(19), 67(93), 57(41), 41(53), 29(31), 27(20)
Geruchsprofil: grün, fruchtig

### 1-Cyclopent-2-en-1-ylpent-4-en-2-one (Verbindung XXIII)

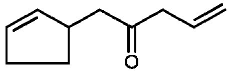

GCMS: m/z (%) = 150(1), 109(42), 81(7), 79(8), 68(6), 67(100), 66(5), 65(6), 53(5), 41(19), 39(10)
Geruchsprofil: grün, fruchtig

### (E)-1-Cyclopent-2-en-1-ylpent-3-en-2-one (Verbindung XXIV)

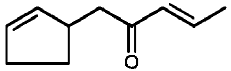

GCMS: m/z (%) = 150(5), 122(16), 107(16), 84(42), 80(26), 79(18), 69(100), 67(43), 66(16), 41(48), 39(22)
Geruchsprofil: grün, fruchtig, etwas eugenolartig

### (Z)-1-Cyclopent-2-en-1-ylpent-3-en-2-one (Verbindung XXV)

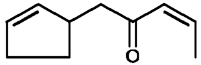

GCMS: m/z (%) = 150(5), 122(16), 107(16), 84(50), 80(27), 79(19), 69(100), 67(32), 66(15), 41(47), 39(23)
Geruchsprofil: grün, fruchtig, etwas eugenolartig

### Beispiel 3

### Geruchsbeschreibung von bevorzugten Riechstoffen nach Zusatz von Verbindungen der Formel (I) bzw. (Ia)

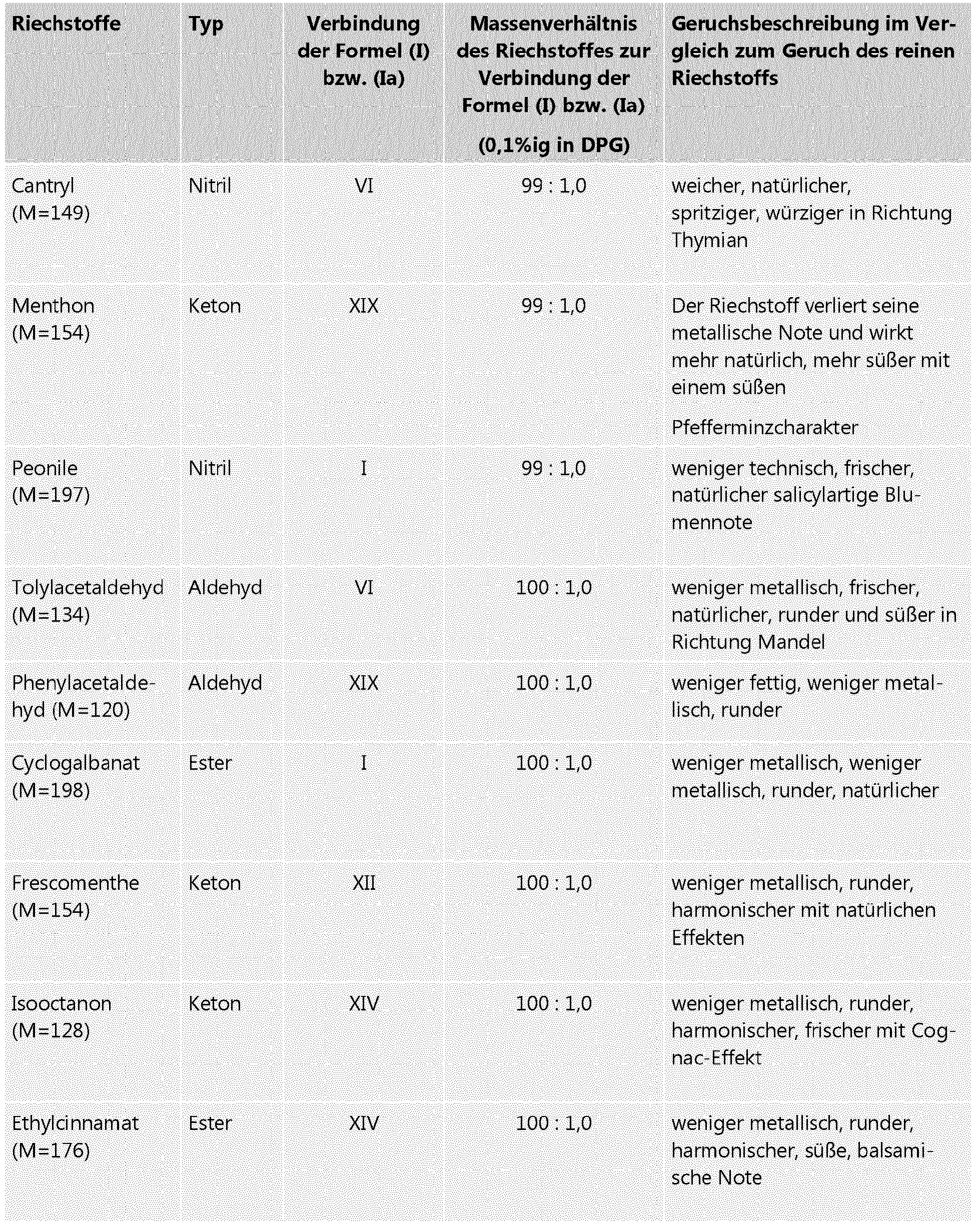

**Fazit:** Die Verbindungen der Formel (I) bzw. (Ia) fördern den natürlichen spritzigen Effekt in kautigen, minzigen und grünen Riechstoffen.

### Beispiel 4

### Parfümöle P1 -P5

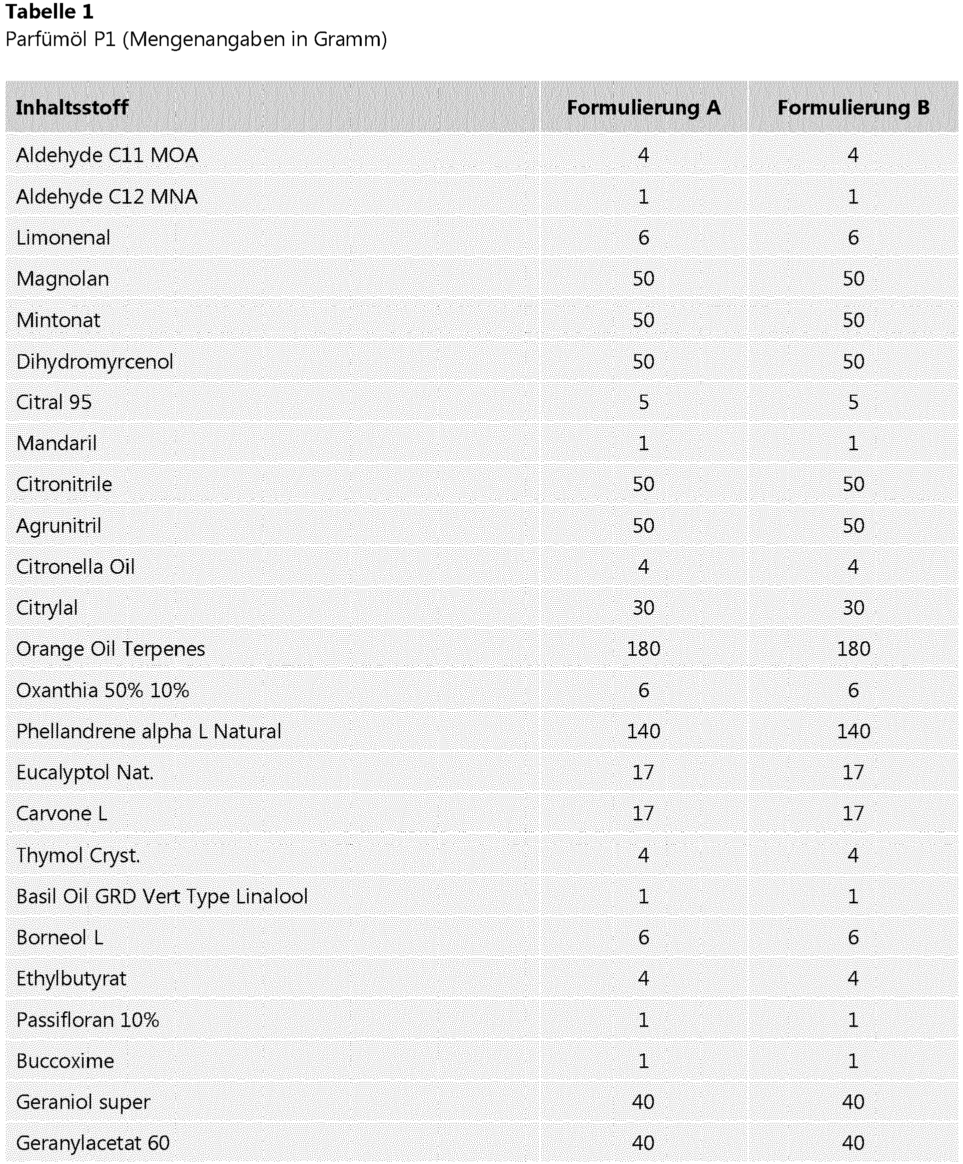

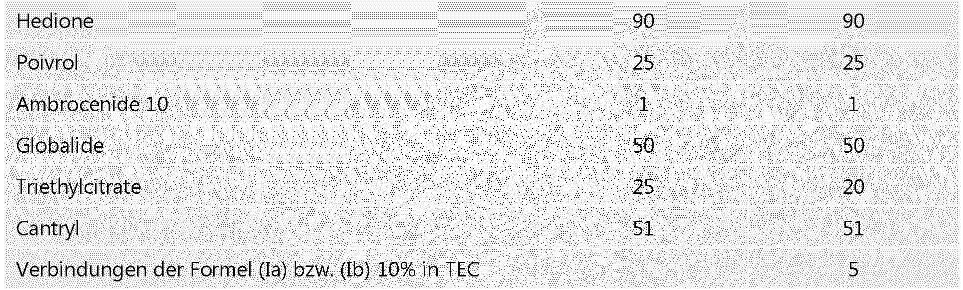

Version B ist deutlich spritziger in der Frische und rundet die blumige Note fein ab. Die ausgeprägte Seifennote in Version A wird durch die Verbindungen der Formel (I) bzw. (Ia) ausgeglichen. Besonders deutlich wird der Efekt mit den Verbindungen VII und XVI beurteilt.

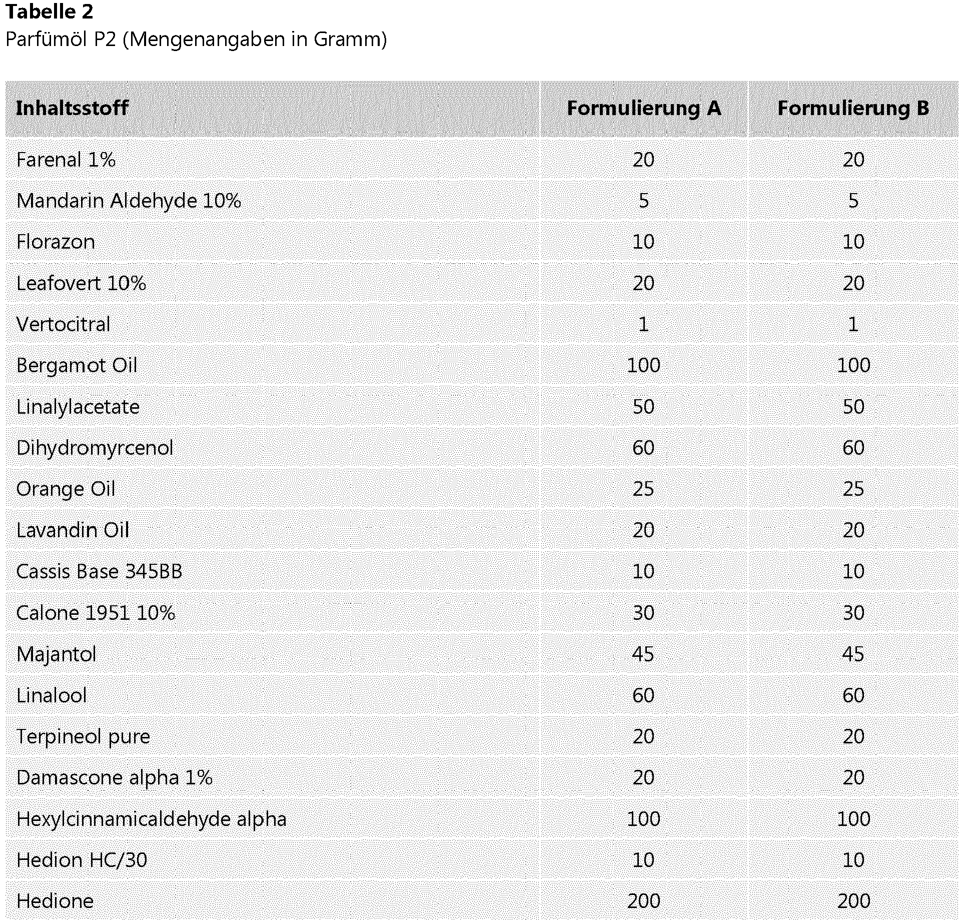

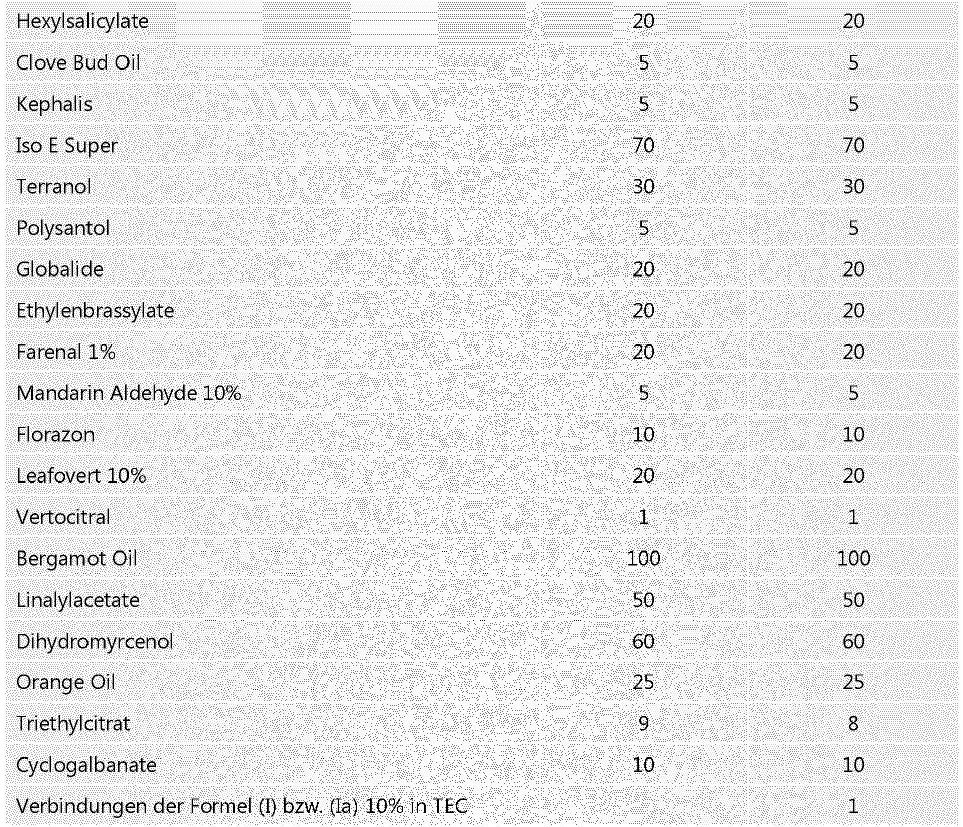

Durch Version B wird die aggressive grüne Kopfnote genommen. Die marinigen Ackorde werden durch Zugabe der Verbindungen der Formel (I) bzw. (Ia) in den Duft integriert und es entsteht ein leichter Fichteneffekt. Besonders deutlich wird der Efekt mit den Verbindungen II und XXII beurteilt.

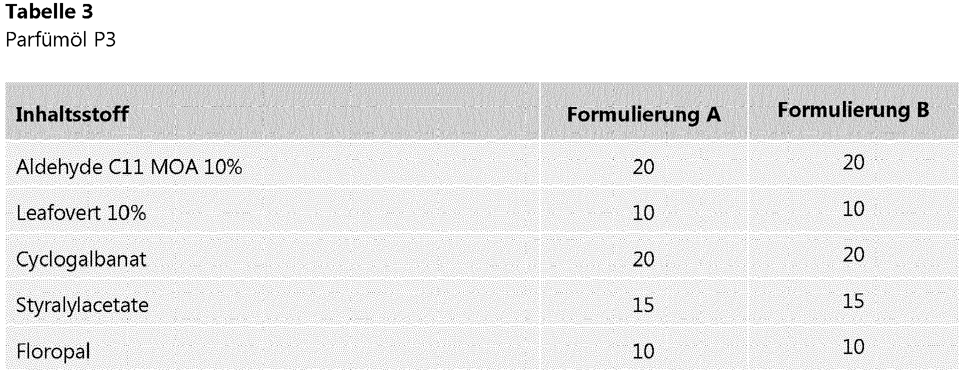

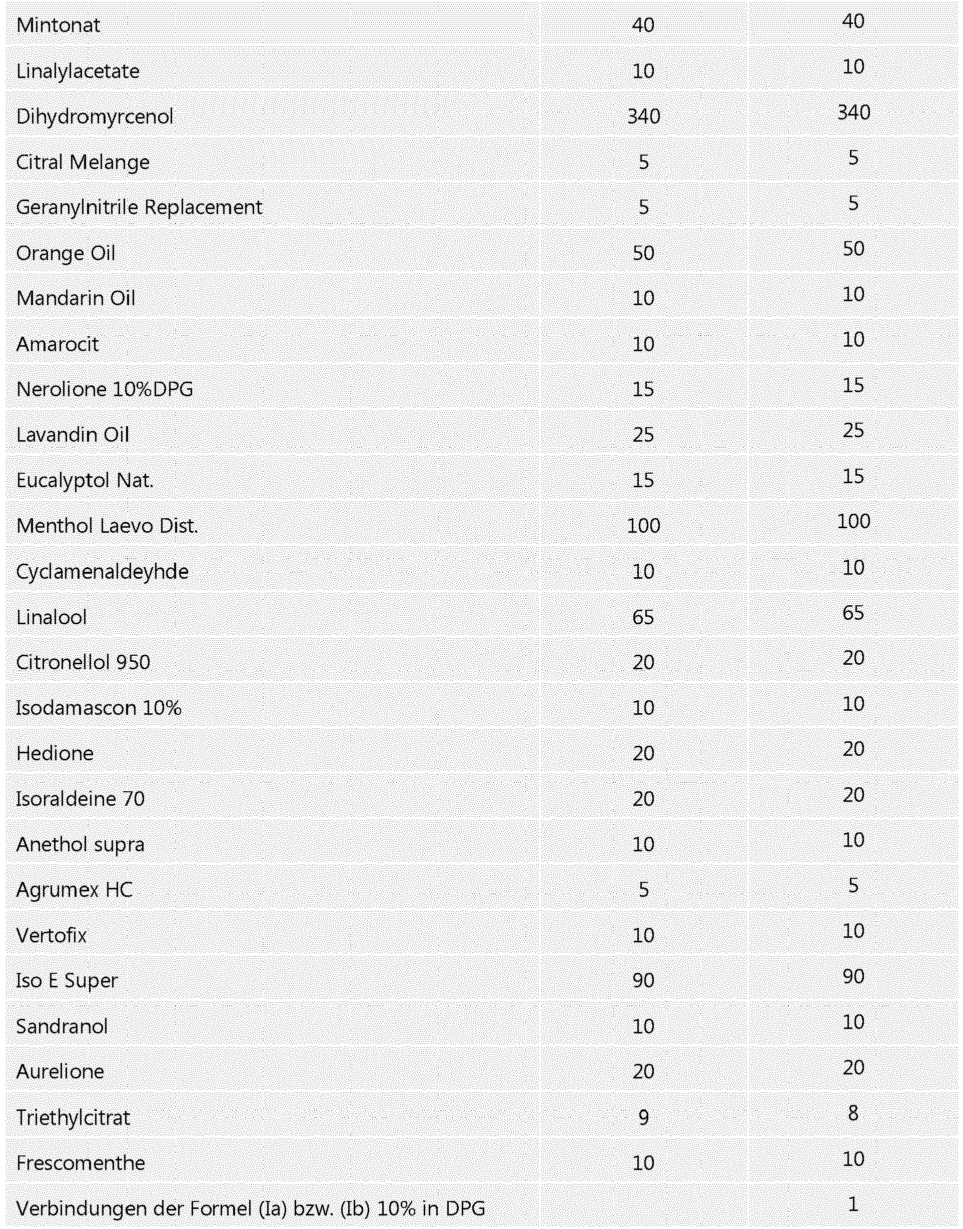

Die Verbindungen der Formel (I) bzw. (Ia) verstärken die frischen und minzigen Noten. Ein transparenter, wässriger Effekt entsteht und lässt die Komposition sauberer wirken. Besonders deutlich wird der Efekt mit den Verbindungen XIV und XXIII beurteilt.

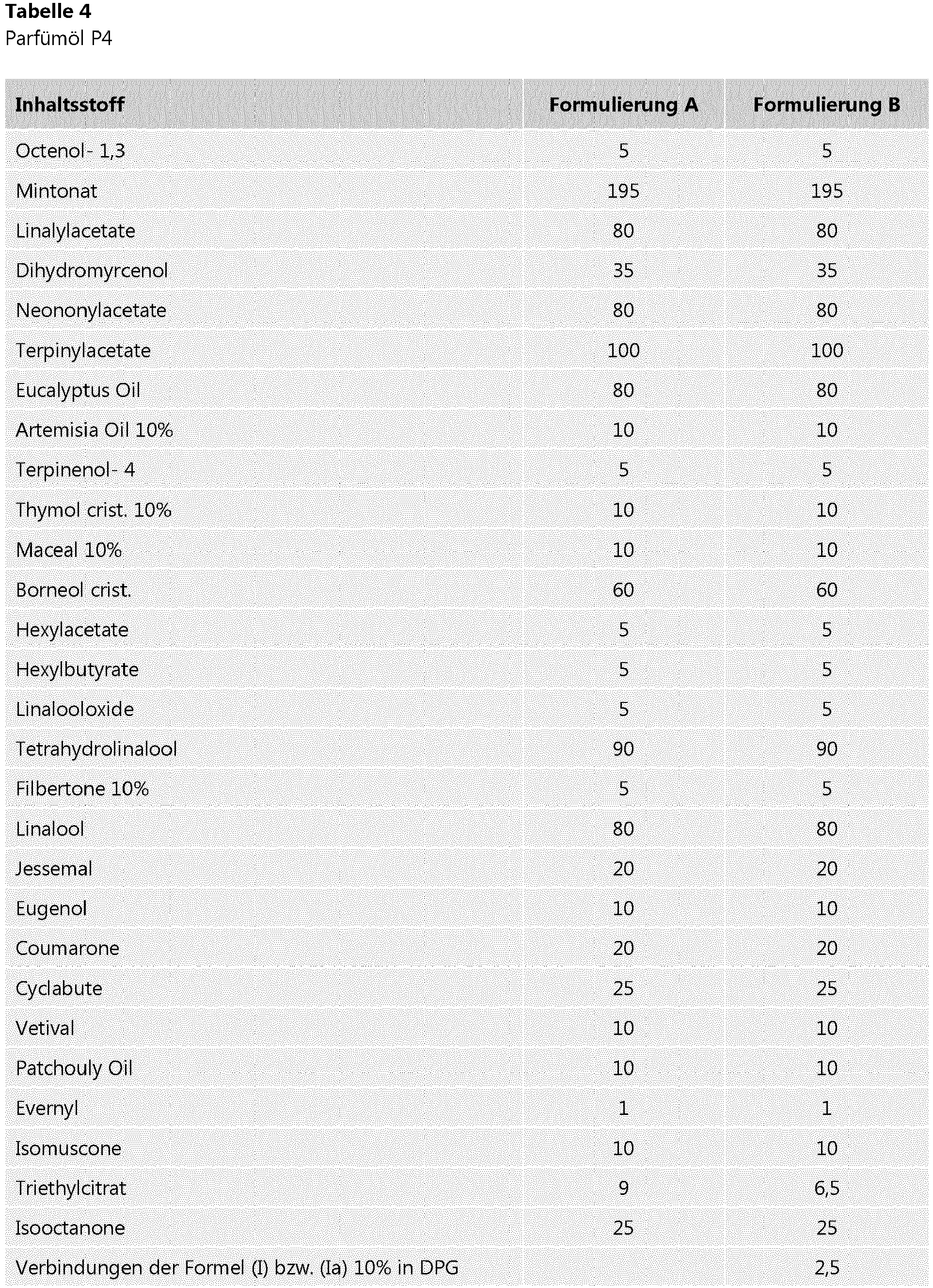

Hier drehen die Verbindungen der Formel (I) bzw. (Ia) den Duft in eine krautige Richtung. Ein süßer Eindruck entsteht, dessen Lavendel-Cumarin Note ausgeprägter erscheint. Gleichzeitig wird die kampfrigeNote ein wenig abgedeckt. Besonders deutlich wird der Efekt mit den Verbindungen XV und XXII beurteilt.

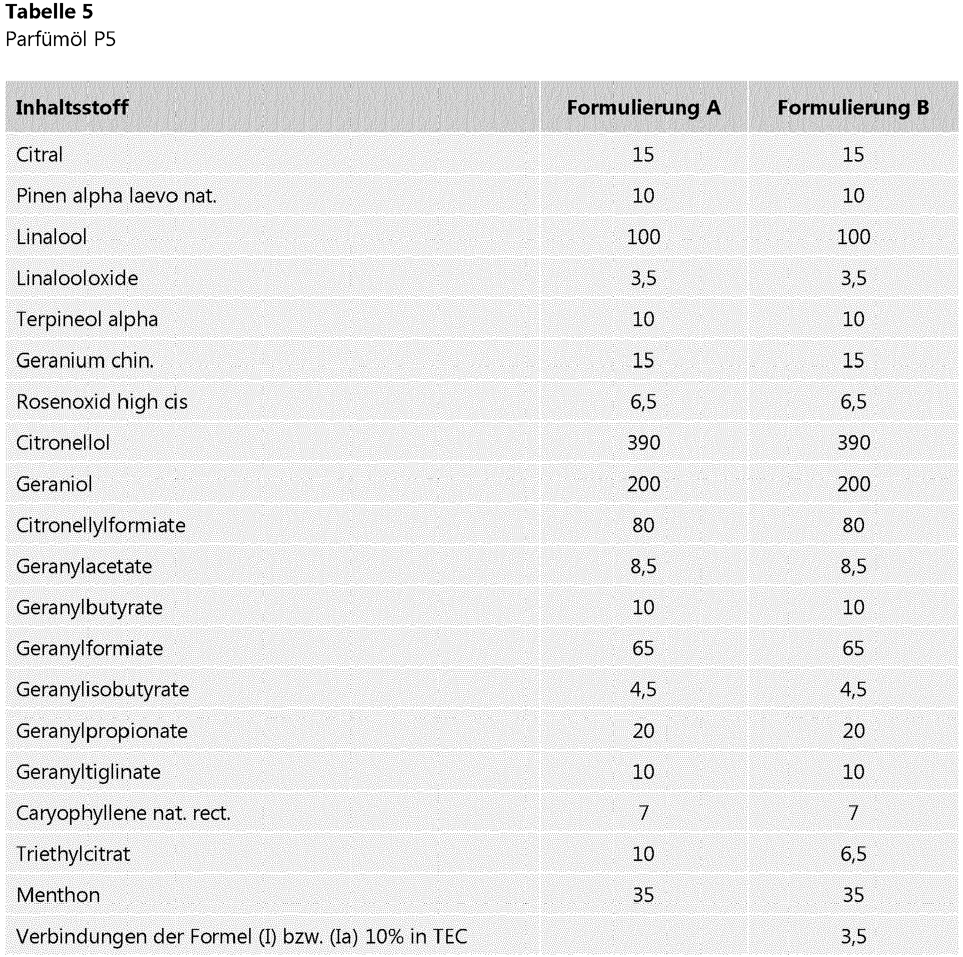

Durch die Verbindungen der Formel (I) bzw. (Ia) wird auch hier die Frische gepuscht. Die leicht metallische Minznote wird deutlich reduziert und abgerundet. Besonders deutlich wird der Efekt mit den Verbindungen XX und XXI beurteilt.

### Beispiel 5

### Formulierungsbeispiele F1 bis F11

Die Parfumöle P1, P2, P3, P4 bzw. P5 aus den obigen Parfümöl-Beispielen 1 bis 5 wurden jeweils separat in die nachfolgenden Formulierungen eingearbeitet. Die oben bei dem jeweiligen Parfümöl beschriebenen geruchlichen Effekte wurden jeweils auch in den nachfolgenden Formulierungen beobachtet.

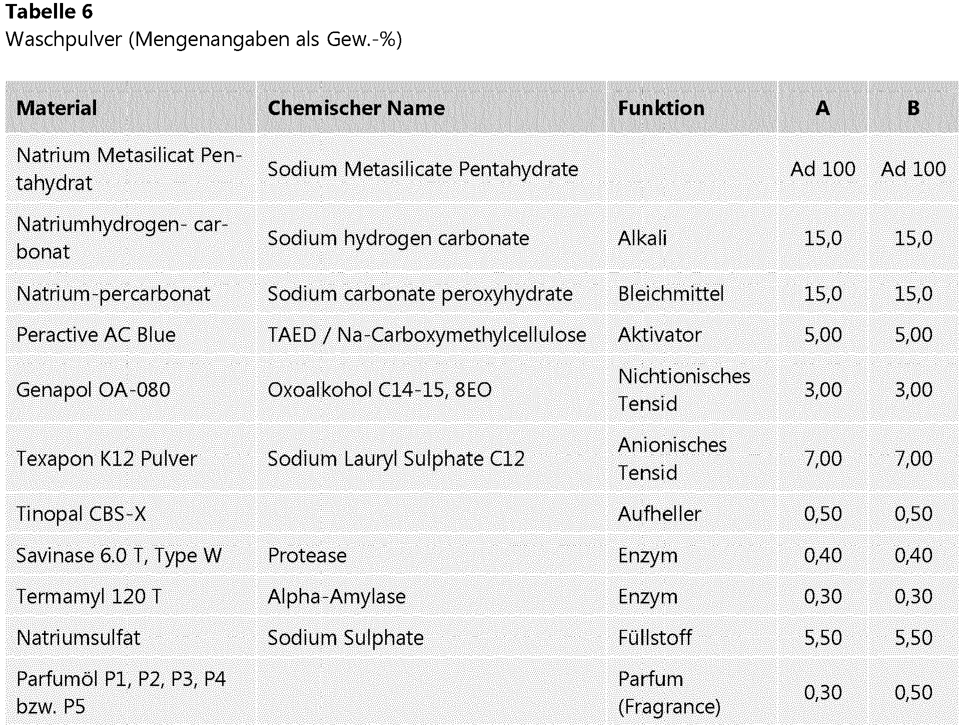

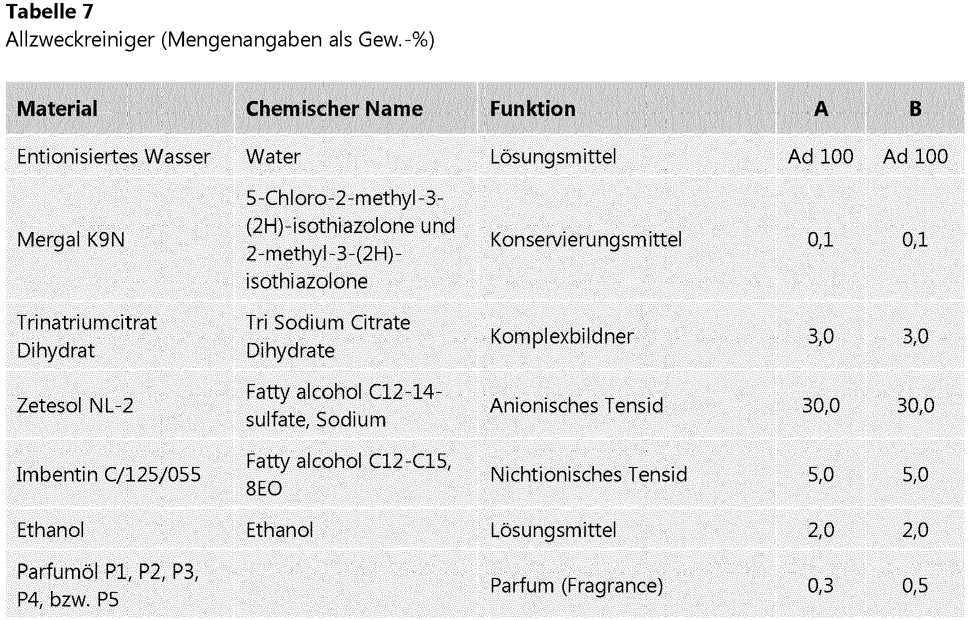

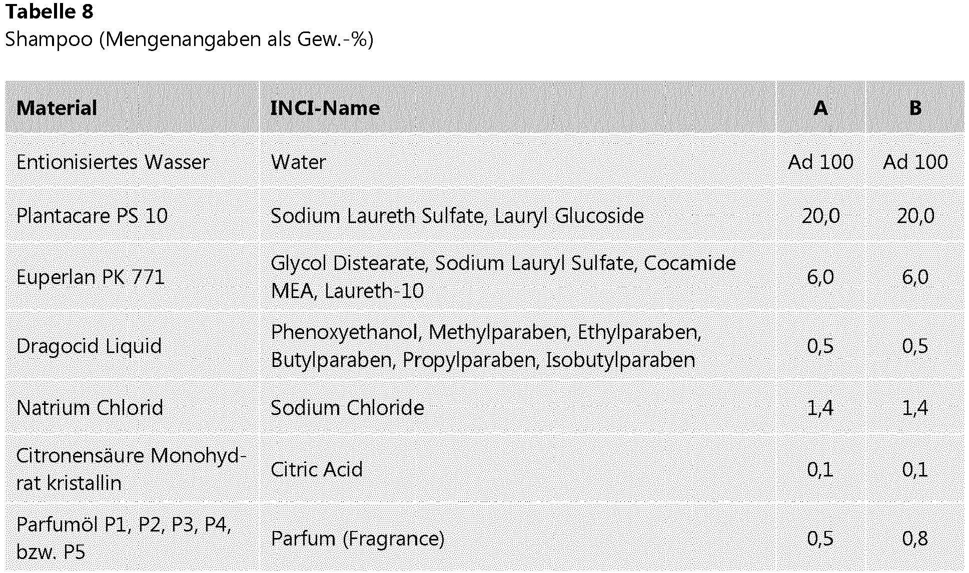

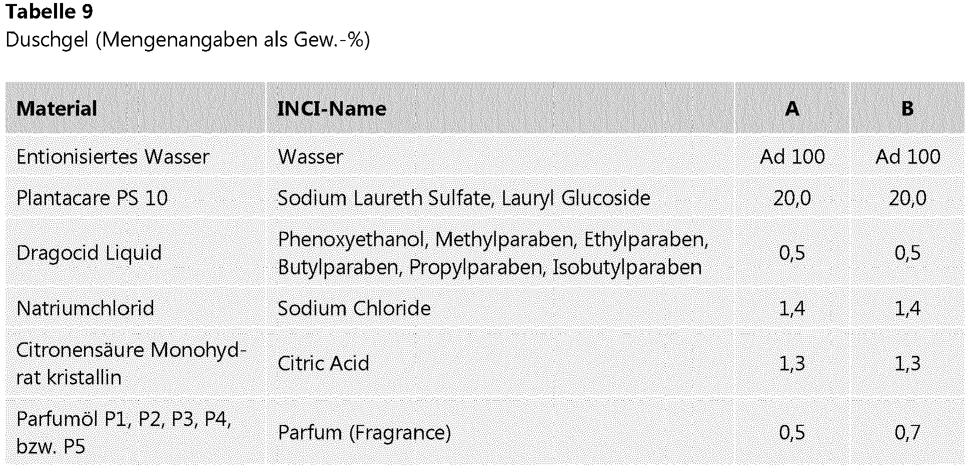

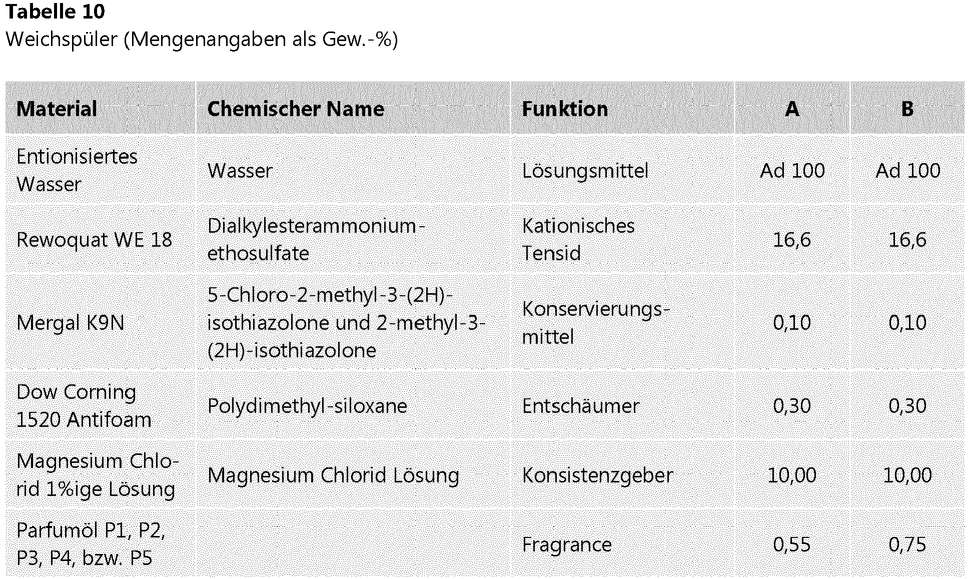

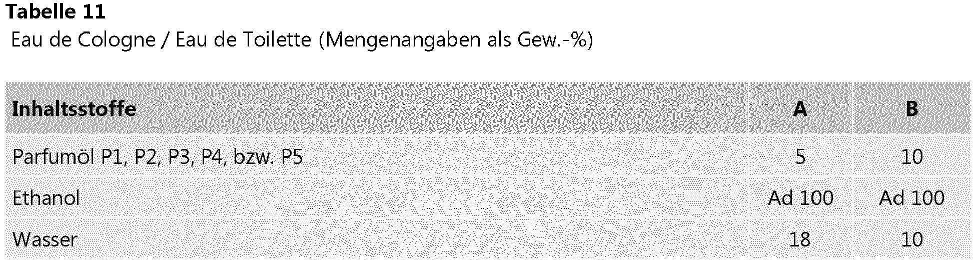

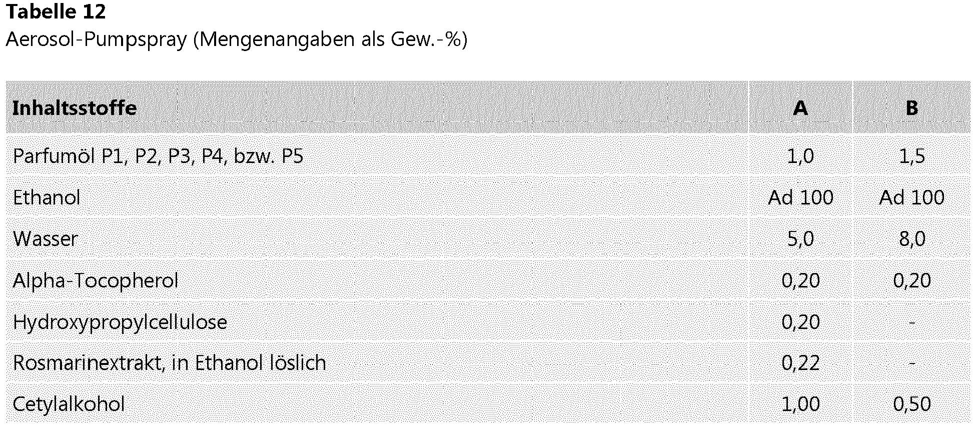

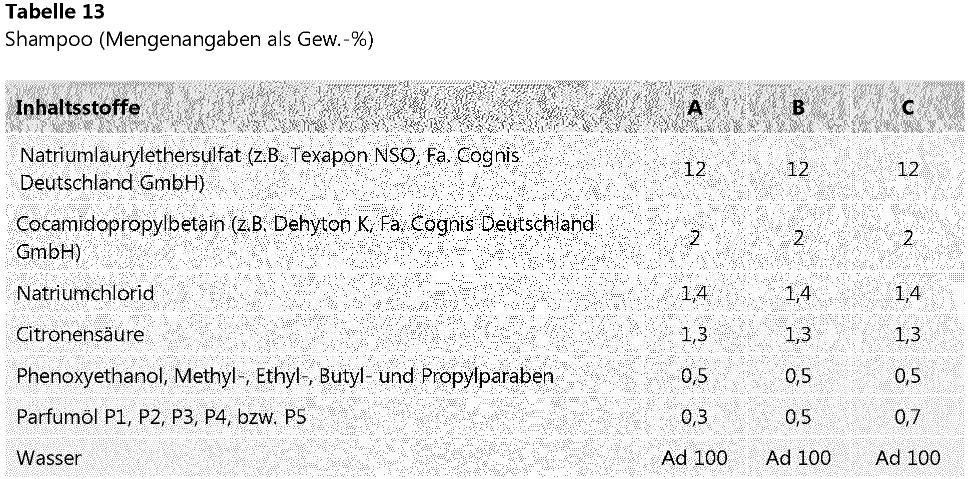

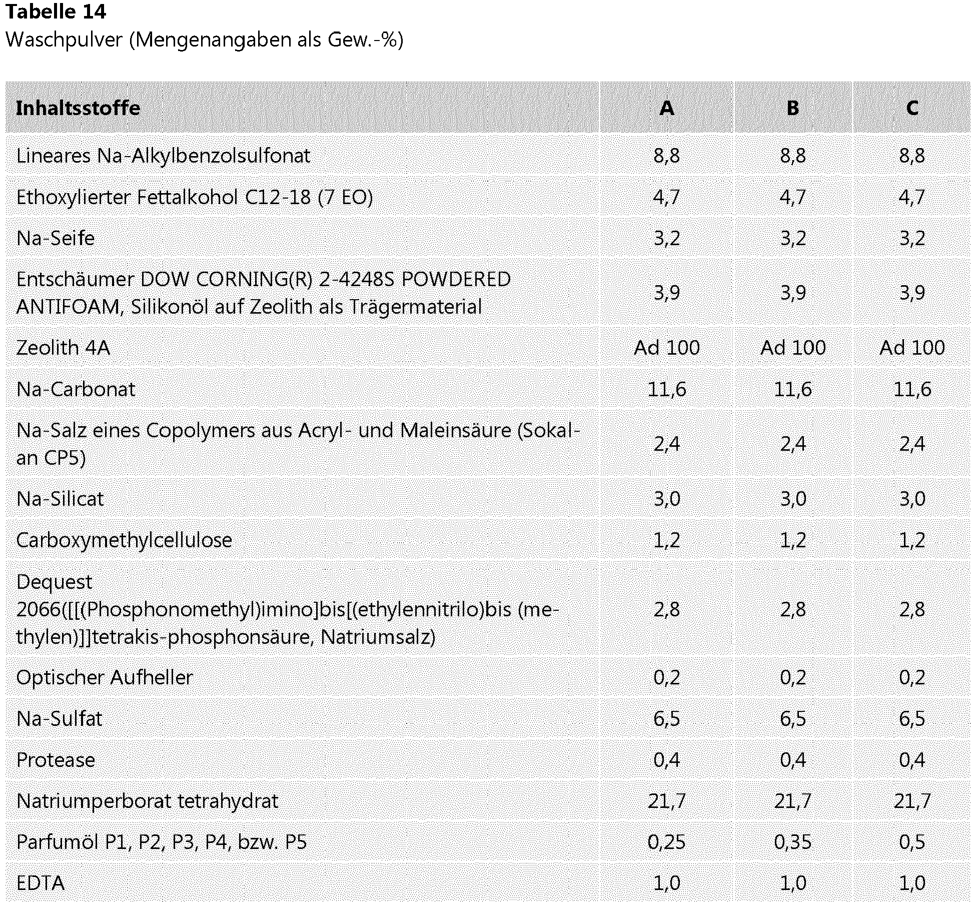

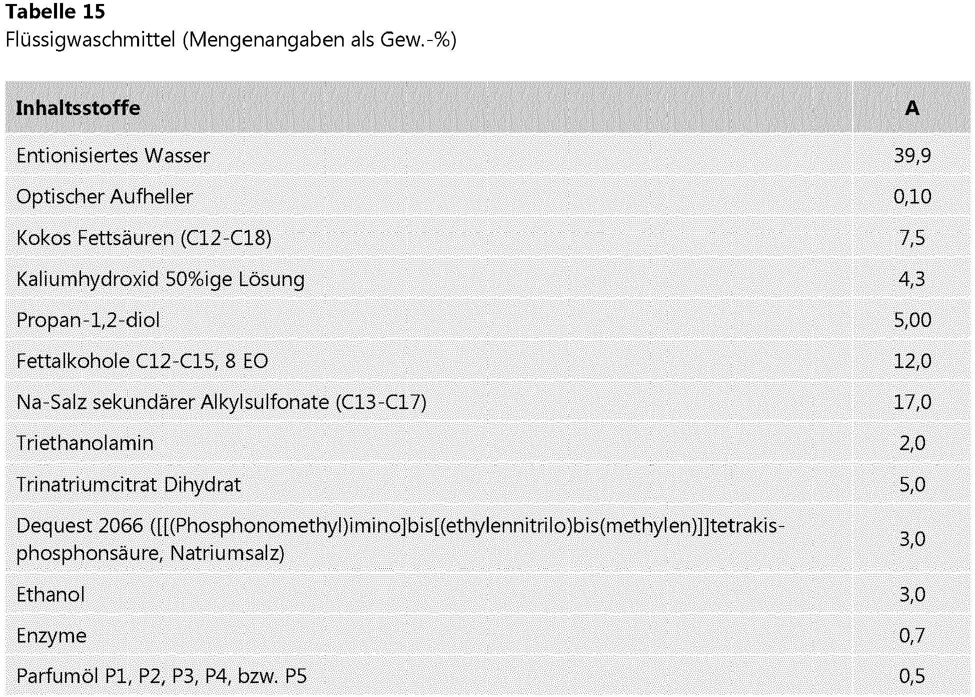

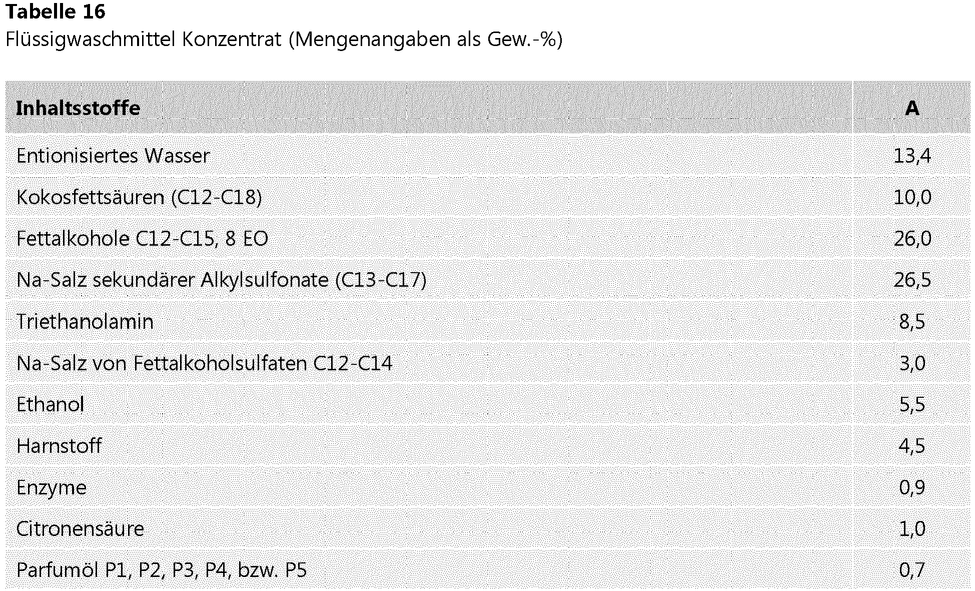

### Beispiel 6

### Stabilitätstest

Der Weichsprüler F5 und Eau de Cologne F6 aus Beispiel 5 wurden gelagert um die Stabilität der Formulierung bzw. die Dufnote nach der Lagerungszeit zu bewerten. Die Ergebnisse sind in **Tabelle 17** wiedergegeben:

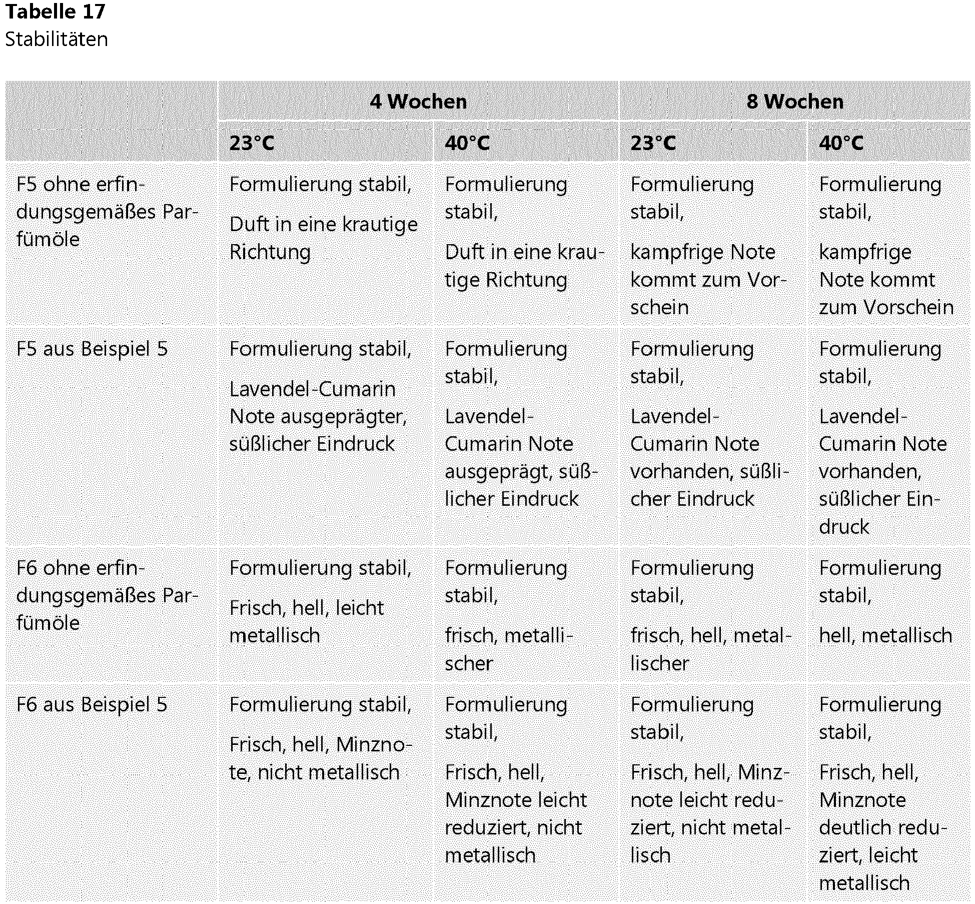

## Patentansprüche

1. Riechstoffmischungen, umfassend mindestens eine Verbindung
(a) der allgemeinen Formel (I): 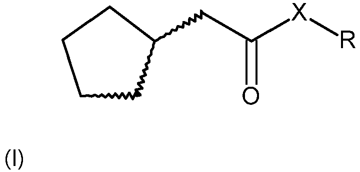 wobei für die Verbindung der Formel (I) bzw. jede Verbindung der Formel (I) gilt, dass X ein Sauerstoffatom oder eine Methylengruppe ist, mit den Maßgaben, dass
(i) keine oder eine der drei gewellten Linien eine Doppelbindung bedeutet bzw. bedeuten und die übrigen gewellten Linien jeweils eine Einfachbindung darstellen, und
(ii) R für einen linearen oder verzweigten, cyclischen, gesättigten oder ungesättigten Kohlenwasserstoffrest mit 1 bis 5 Kohlenstoffatomen steht, und
(b) einen oder mehrere weitere Riechstoffe enthalten, die ausgewählt sind aus der Gruppe, die gebildet wird von Cantryl, Peonile, Menthon, Frescomenthe, Isooctanon, Tolylacetaldehyd, Phenylacetaldehyd, Cyclogalbanat, Ethylcinnamat oder Mischungen daraus.

2. Riechstoffmischungen nach Anspruch 1, **dadurch gekennzeichnet, dass** die Verbindung (a) der allgemeinen Formel (I) ausgewählt ist aus einer Verbindung der Formel (la) oder (Ib), oder Mischungen daraus: 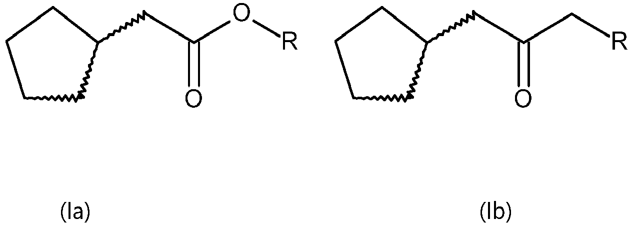 wobei für die Verbindung der Formel (la) oder (Ib) bzw. jede Verbindung der Formel (la) oder (Ib) gilt, dass
(i) keine oder eine der drei gewellten Linien eine Doppelbindung bedeutet bzw. bedeuten und die übrigen gewellten Linien jeweils eine Einfachbindung darstellen, und
(ii) R für einen linearen oder verzweigten, cyclischen, gesättigten oder ungesättigten Kohlenwasserstoffrest mit 1 bis 5 Kohlenstoffatomen steht.

3. Riechstoffmischungen nach mindestens einem der vorhergehenden Ansprüche 1 und/oder 2, **dadurch gekennzeichnet, dass** sie ferner einen oder mehrere weitere Riechstoffe (c) enthalten, welche als Fondnote fungieren und ausgewählt sind aus der Gruppe bestehend aus Ketonen und Estern oder Lactonen mit einer Molmasse im Bereich von 220 g/mol bis 320 g/mol.

4. Riechstoffmischungen nach mindestens einem der Ansprüche 1 bis 3, **dadurch gekennzeichnet, dass** das
(i) Massenverhältnis der Gesamtmenge an Riechstoffen (b) zur Verbindung(en) (a) größer oder gleich 99 : 1 ist, und/oder
(ii) das Massenverhältnis der Gesamtmenge an Riechstoffen (c) zur Verbindung (a) größer oder gleich 99 : 1 ist,
jeweils bezogen auf die Gesamtmenge aller Riechstoffe in den einzelnen Verbindungsgruppen (a), (b) und (c), bezogen auf die gesamte Riechstoffmischung.

5. Riechstoffmischungen nach mindestens einem der vorhergehenden Ansprüche 1 bis 4, **dadurch gekennzeichnet, dass** die Verbindungen der Formel (I) bzw. Formel (la) und/oder (Ib) ausgewählt sind aus der Gruppe bestehend aus:
Methyl 2-cyclopent-2-en-1-ylacetate (Verbindung II);
Ethyl 2-cyclopent-2-en-1-ylacetate (Verbindung III);
Propyl 2-cyclopent-2-en-1-ylacetate (Verbindung IV);
Isopropyl 2-cyclopent-2-en-1-ylacetate (Verbindung V);
Allyl 2-cyclopent-2-en-1-ylacetate (Verbindung VI);
Isopentyl 2-cyclopent-2-en-1-ylacetate (Verbindung VII);
Cyclopentyl 2-cyclopent-2-en-1-ylacetate (Verbindung VIII);
[(E)-But-2-enyl] 2-cyclopent-2-en-1-ylacetate (Verbindung IX);
Cyclopropylmethyl 2-cyclopent-2-en-1-ylacetate (Verbindung X);
1,2-Dimethylpropyl 2-cyclopent-2-en-1-ylacetate (Verbindung XI);
Ethyl 2-(cyclopenten-1-yl) acetate (Verbindung XII);
Methyl 2-cyclopentylideneacetate(Verbindung XIII;)
Methyl 2-cyclopentylacetate (Verbindung XIV);
Propyl 2-cyclopentylacetate (Verbindung XV);
Allyl 2-cyclopentylacetate (Verbindung XVI);
Isopropyl 2-cyclopentylacetate (Verbindung XVII);
[(E)-But-2-enyl] 2-cyclopentylacetate (Verbindung XVIII);
3-Methylbut-2-enyl 2-cyclopent-2-en-1-ylacetate (Verbindung XIX);
Ethyl 2-cyclopentylacetate (Verbindung XX);
1-Cyclopent-2-en-1-ylpropan-2-one (Verbindung XXI);
1-Cyclopent-2-en-1-ylhexan-2-one (Verbindung XXII);
1-Cyclopent-2-en-1-ylpent-4-en-2-one (Verbindung XXIII);
(E)-1-Cyclopent-2-en-1-ylpent-3-en-2-one (Verbindung XXIV);
(Z)-1-Cyclopent-2-en-1-ylpent-3-en-2-one (Verbindung XXV).

6. Verwendung von Mischungen nach einem der Ansprüche 1 bis 5 als Parfümöl.

7. Verwendung einer Riechstoffmischung nach mindestens einem der vorhergehenden Ansprüche 1 bis 5 zur Erzeugung, Vermittlung, Modifizierung oder Verstärkung eines fruchtigen Geruchs in Richtung Apfel, Birne, Ananas, Banane und Erdbeere.

8. Verfahren zur Herstellung einer Riechstoffmischung, umfassend die folgenden Schritte:
(a) Bereitstellen einer Verbindung der allgemeinen Formel (I) 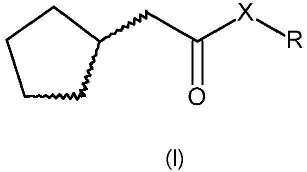 wobei für die Verbindung der Formel (I) bzw. jede Verbindung der Formel (I) gilt, dass X ein Sauerstoffatom oder eine Methylengruppe ist, mit den Maßgaben, dass
(i) keine oder eine der drei gewellten Linien eine Doppelbindung bedeutet bzw. bedeuten und die übrigen gewellten Linien jeweils eine Einfachbindung darstellen, und
(ii) R für einen linearen oder verzweigten, cyclischen, gesättigten oder ungesättigten Kohlenwasserstoffrest mit 1 bis 5 Kohlenstoffatomen steht, und
(b) Bereitstellen mindestens eines weiteren Riechstoffs aus der Gruppe, die gebildet wird von Cantryl, Peonile, Menthon, Frescomenthe, Isooctanon, Tolylacetaldehyd, Phenylacetaldehyd, Cyclogalbanat, Ethylcinnamat oder Mischungen daraus, sowie gegebenenfalls
(c) Bereitstellen mindestens eines oder mehrerer weiterer Riechstoffe ausgewählt aus der Gruppe bestehend aus Ketonen und Estern oder Lactonen mit einer Molmasse im Bereich von 220 g/mol bis 320 g/mol, sowie
(d) Vermischen der Bestandteile (a), (b) und gegebenenfalls (c).

9. Verfahren zum Verstärken der natürlichen Frische und/oder Ausstrahlung und/oder zum Maskieren oder Vermindern fettiger und/oder metallischer Noten eines oder mehrerer Verbindung(en) der von der allgemeinen Formel (I) verschiedenen Riechstoffe, umfassend den folgenden Schritt:
Vermischen von Riechstoffen, die ausgewählt sind aus den Gruppen, die gebildet werden von
(b) Cantryl, Peonile, Menthon, Frescomenthe, Isooctanon, Tolylacetaldehyd, Phenylacetaldehyd, Cyclogalbanat, Ethylcinnamat oder Mischungen daraus und /oder
(c) einen oder mehreren weiteren Riechstoffe ausgewählt aus der Gruppe bestehend aus Ketonen und Estern oder Lactonen mit einer Molmasse im Bereich von 220 g/mol bis 320 g/mol
mit einer Menge an Verbindung der Formel (I), die ausreicht, die natürliche Frische und/oder Ausstrahlung des oder der von Verbindung(en) der allgemeinen Formel (I) verschiedener Riechstoffe [zu verstärken und/oder fettige und/oder metallische Noten zu maskieren oder zu vermindern.

10. Verfahren nach Anspruch 9, wobei man
(i) das Massenverhältnis der Gesamtmenge an Riechstoffen b) zur Verbindung der Formel (I) größer oder gleich 99:1 ist, und/oder
(ii) das Massenverhältnis der Gesamtmenge an Riechstoffen c) zur Verbindung der Formel (I) größer oder gleich 99:1
einstellt.

11. Parfümiertes Produkt enthaltend eine Riechstoffmischung, vorzugsweise ein Parfümöl, nach einem der Ansprüche 1 bis 5, in einer sensorisch wirksamen Menge.

12. Parfümiertes Produkt nach Anspruch 11, wobei das Produkt ausgewählt ist aus der Gruppe bestehend aus:
Parfüm-Extraits, Eau de Parfums, Eau de Toilettes, Rasierwässer, Eau de Colognes, Pre-shave-Produkte, Splash-Colognes, parfümierte Erfrischungstücher, saure, alkalische und neutrale Reinigungsmittel, Textilerfrischer, Bügelhilfen, flüssige Waschmittel, pulverförmige Waschmittel, Wäschevorbehandlungsmittel, Wäscheweichspüler, Waschseifen, Waschtabletten, Desinfektionsmittel, Oberflächendesinfektionsmittel, Luftverbesserer, Aerosolsprays, Wachse und Polituren, Körperpflegemittel, Handcremes und -lotionen, Fußcremes und -lotionen, Enthaarungscremes und -lotionen, After-shave-Cremes und -lotionen, Bräunungscremes und -lotionen, Haarpflegeprodukten, Deodorantien und Antiperspirantien, Produkte der dekorativen Kosmetik, Kerzen, Lampenöle, Räucherstäbchen, Insektizide, Repellentien und Treibstoffe.

13. Parfümiertes Produkt nach den Ansprüchen 11 oder 12, **dadurch gekennzeichnet**, wobei der Anteil der Riechstoffmischung nach einem der Ansprüche 1 bis 5 an dem parfümierten Produkt 0,01 bis 10 Gew.-% beträgt, bezogen auf die Gesamtmasse des parfümierten Produktes.

## Claims

1. Perfume mixtures comprising
(a) at least one compound of the general formula (I): 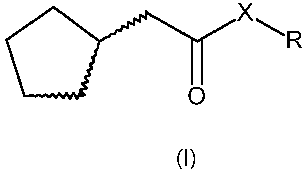 wherein the or each compound of formula (I) is that X is an oxygen atom or a methylene group, with the provisos that
(i) none or one of the three wavy lines represents a double bond and the remaining wavy lines each represent a single bond, and
(ii) R represents a linear or branched, cyclic, saturated or unsaturated hydrocarbon radical having 1 to 5 carbon atoms, and
(b) contain one or more further perfumes selected from the group formed by cantryl, peonile, menthone, frescomenthe, isooctanone, tolylacetaldehyde, phenylacetaldehyde, cyclogalbanate, ethyl cinnamate or mixtures thereof.

2. Perfume mixtures according to claim 1, **characterized in that** compound (a) of the general formula (I) is selected from a compound of the formula (la) or (lb), or mixtures thereof: 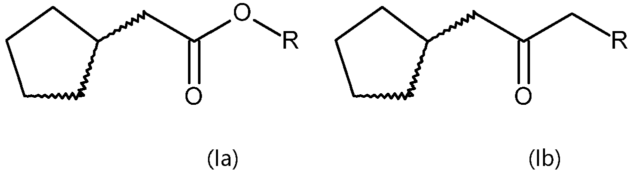 wherein for the compound of formula (la) or (lb), or any compound of formula (la) or (lb), it is the case that
(i) none or one of the three wavy lines represents a double bond and the remaining wavy lines each represent a single bond; and
(ii) R represents a linear or branched, cyclic, saturated or unsaturated hydrocarbon radical having 1 to 5 carbon atoms.

3. Perfume mixtures according to at least one of the preceding claims 1 and/or 2, **characterized in that** they further contain one or more further perfumes (c) which act as a fond note and are selected from the group consisting of ketones and esters or lactones having a molar mass in the range from 220 g/mol to 320 g/mol.

4. Perfume mixtures according to at least one of claims 1 to 3, **characterized in that** the
(i) mass ratio of the total amount of perfumes (b) to the compound(s) (a) is greater than or equal to 99:1, and/or
(ii) the mass ratio of the total amount of odorants (c) to the compound(s) (a) is greater than or equal to 99:1,
in each case based on the total amount of all perfumes in the individual compound groups (a), (b) and (c), relative to the total perfume mixture.

5. Perfume mixtures according to at least one of the preceding claims 1 to 4, **characterized in that** the compounds of formula (I) or formula (la) and/or (lb) are selected from the group consisting of:
Methyl 2-cyclopent-2-en-1-ylacetate (compound II);
Ethyl 2-cyclopent-2-en-1-ylacetate (compound III);
Propyl 2-cyclopent-2-en-1-ylacetate (compound IV);
Isopropyl 2-cyclopent-2-en-1-ylacetate (Compound V);
Allyl 2-cyclopent-2-en-1-ylacetate (Compound VI);
Isopentyl 2-cyclopent-2-en-1-ylacetate (Compound VII);
Cyclopentyl 2-cyclopent-2-en-1-ylacetate (Compound VIII);
[(E)-but-2-enyl] 2-cyclopent-2-en-1-ylacetate (Compound IX);
Cyclopropylmethyl 2-cyclopent-2-en-1-ylacetate (compound X);
1,2-dimethylpropyl 2-cyclopent-2-en-1-ylacetate (compound XI);
Ethyl 2-(cyclopenten-1-yl) acetate (compound XII);
Methyl 2-cyclopentylidene acetate(Compound XIII;)
Methyl 2-cyclopentylacetate (Compound XIV);
Propyl 2-cyclopentylacetate (Compound XV);
Allyl 2-cyclopentylacetate (Compound XVI);
Isopropyl 2-cyclopentylacetate (Compound XVII);
[(E)-but-2-enyl] 2-cyclopentylacetate (Compound XVIII);
3-methylbut-2-enyl 2-cyclopent-2-ene-1-yl acetate (Compound XIX);
Ethyl 2-cyclopentylacetate (Compound XX);
1-Cyclopent-2-en-1-ylpropan-2-one (Compound XXI);
1-Cyclopent-2-en-1-ylhexan-2-one (Compound XXII);
1-cyclopent-2-en-1-ylpent-4-en-2-ones (compound XXIII);
(E)-1-cyclopent-2-en-1-ylpent-3-en-2-ones (compound XXIV);
(Z)-1-cyclopent-2-en-1-ylpent-3-en-2-ones (compound XXV).

6. Use of mixtures according to any one of claims 1 to 5 as perfume oil.

7. Use of a perfume blend according to at least one of the preceding claims 1 to 5 for producing, imparting, modifying or enhancing a fruity odor in the direction of apple, pear, pineapple, banana and strawberry.

8. A method of preparing a fragrance blend comprising the steps of:
(a) providing a compound of the general formula (I). 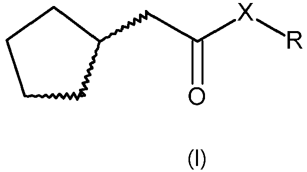 wherein the or each compound of formula (I) is that X is an oxygen atom or a methylene group, with the provisos that
(i) none or one of the three wavy lines represents a double bond and the remaining wavy lines each represent a single bond, and
(ii) R represents a linear or branched, cyclic, saturated or unsaturated hydrocarbon radical having 1 to 5 carbon atoms, and
(b) providing at least one other fragrance selected from the group consisting of cantryl, peonile, menthone, frescomenthe, isooctanone, tolylacetaldehyde, phenylacetaldehyde, cyclogalbanate, ethyl cinnamate or mixtures thereof, and optionally
(c) providing at least one or more further perfumes selected from the group consisting of ketones and esters or lactones having a molecular weight in the range of 220 g/mol to 320 g/mol, and
(d) mixing components (a), (b) and optionally (c).

9. A process for enhancing the natural freshness and/or radiance and/or masking or reducing greasy and/or metallic notes of one or more compound(s) of the perfumes different from the general formula (I), comprising the following step:
Mixing perfumes selected from the groups formed by.
(b) cantryl, peonile, menthone, frescomenthe, isooctanone, tolylacetaldehyde, phenylacetaldehyde, cyclogalbanate, ethylcinnamate or mixtures thereof, and/or
(c) one or more further perfumes selected from the group consisting of ketones and esters or lactones having a molecular weight in the range of 220 g/mol to 320 g/mol with an amount of compound of formula (I) sufficient to enhance the natural freshness and/or radiance of the fragrance(s) other than compound(s) of general formula (I) [and/or to mask or reduce greasy and/or metallic notes.

10. The process according to claim 9, wherein.
(i) the mass ratio of the total amount of perfumes b) to the compound of formula (I) is greater than or equal to 99:1, and/or
(ii) the mass ratio of the total amount of perfumes c) to the compound of formula (I) being greater than or equal to 99:1
is adjusted.

11. A perfumed product comprising a perfume mixture, preferably a perfume oil, according to any one of claims 1 to 5, in a sensory effective amount.

12. The perfumed product according to claim 11, wherein the product is selected from the group consisting of:
Perfume extracts, eau de parfums, eau de toilettes, shaving waters, eau de colognes, pre-shave products, splash colognes, perfumed refreshing wipes, acid, alkaline and neutral detergents, fabric fresheners, ironing aids, liquid detergents, powder detergents, laundry pre-treatment agents, fabric softeners, laundry soaps, laundry tablets, disinfectants, surface disinfectants, air fresheners, aerosol sprays, waxes and polishes, personal care products, hand creams and lotions, foot creams and lotions, depilatory creams and lotions, after-shave creams and lotions, tanning creams and lotions, hair care products, deodorants and antiperspirants, decorative cosmetic products, candles, lamp oils, incense sticks, insecticides, repellents and propellants.

13. The perfumed product according to claims 11 or 12, **characterized in that** the proportion of the perfume mixture according to any one of claims 1 to 5 in the perfumed product is 0.01 to 10% by weight, based on the total mass of the perfumed product.

## Revendications

1. Mélanges de parfums comprenant
(a) au moins un composé de formule générale (I) : 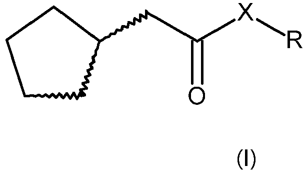 dans laquelle, pour le composé de formule (I) ou chaque composé de formule (I), X est un atome d'oxygène ou un groupe méthylène, à condition que
(i) aucune ou une des trois lignes ondulées ne représente une double liaison et les lignes ondulées restantes représentent chacune une simple liaison, et que
(ii) R représente un radical hydrocarboné linéaire ou ramifié, cyclique, saturé ou insaturé, comportant de 1 à 5 atomes de carbone, et
(b) contiennent un ou plusieurs autres parfums choisis dans le groupe formé par le cantryle, le péonile, la menthone, le frescomenthe, l'isooctanone, le tolylacétaldéhyde, le phénylacétaldéhyde, le cyclogalbanate, le cinnamate d'éthyle ou leurs mélanges.

2. Mélanges de parfums selon la revendication 1, **caractérisés en ce que** le composé (a) de formule générale (I) est choisi parmi un composé de formule (la) ou (Ib), ou des mélanges de ceux-ci : 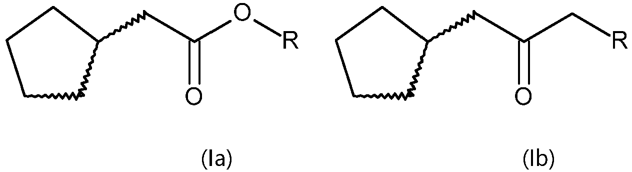 où, pour le composé de formule (la) ou (Ib), ou pour chaque composé de formule (la) ou (Ib), respectivement, on a
(i) aucune ou une des trois lignes ondulées ne représente une double liaison et les lignes ondulées restantes représentent chacune une simple liaison, et que
(ii) R représente un radical hydrocarboné linéaire ou ramifié, cyclique, saturé ou insaturé, comportant de 1 à 5 atomes de carbone.

3. Mélanges de parfums selon au moins l'une des revendications 1 et/ou 2 précédentes, **caractérisés en ce qu'**ils contiennent en outre une ou plusieurs autres substances odorantes (c) qui agissent comme note de fond et sont choisies dans le groupe constitué par les cétones et les esters ou les lactones ayant une masse molaire dans la plage de 220 g/mol à 320 g/mol.

4. Mélanges de parfums selon au moins une des revendications 1 à 3, **caractérisés en ce que**
(i) le rapport massique de la quantité totale de substances odorantes (b) au(x) composé(s) (a) est supérieur ou égal à 99:1, et/ou
(ii) le rapport massique de la quantité totale de substances odorantes (c) au composé (a) est supérieur ou égal à 99:1,
dans chaque cas, par rapport à la quantité totale de toutes les substances odorantes dans les différents groupes de composés (a), (b) et (c), par rapport au mélange total de substances odorantes.

5. Mélanges de parfums selon au moins l'une des revendications précédentes 1 à 4, **caractérisés en ce que** les composés de formule (I) ou de formule (la) et/ou (Ib) sont choisis dans le groupe constitué par :
Méthyl 2-cyclopent-2-ène-1-acétate de yyle (composé II) ;
2-cyclopent-2-ène-1-acétate d'éthyle (composé III) ;
2-cyclopent-2-ène-1-acétate de propyle (composé IV) ;
2-cyclopent-2-ène-1-acétate d'isopropyle (composé V) ;
Allyl 2-cyclopent-2-ène-1-acétate de vinyle (composé VI) ;
Isopentyl 2-cyclopent-2-ène-1-acétate (composé VII) ;
2-cyclopent-2-ène-1-acétate de cyclopentyle (composé VIII) ;
[(E)-but-2-ényl] 2-cyclopent-2-ène-1-acétate de vinyle (composé IX) ;
2-cyclopent-2-ène-1-acétate de cyclopropylméthyle (composé X) ;
2-cyclopent-2-ène-1-acétates de 1,2-diméthylpropyle (composé XI) ;
Acétate d'éthyle 2-(cyclopentène-1-yle) (composé XII) ;
méthyl 2-cyclopentylidène acétate (composé XIII ;)
Acétate de méthyle et de 2-cyclopentyle (composé XIV) ;
Acétate de propyle et de 2-cyclopentyle (composé XV) ;
Allyl 2-cyclopentylacétate (composé XVI) ;
Acétate d'isopropyle-2-cyclopentyle (composé XVII) ;
[(E)-but-2-ényl] acétate de 2-cyclopentyle (composé XVIII) ;
3-méthylbut-2-ényl 2-cyclopent-2-ène-1-acétate de yttrium (composé XIX) ;
Acétate d'éthyle-2-cyclopentyle (composé XX) ;
1-cyclopent-2-ène-1-ylpropan-2-one (composé XXI) ;
1-cyclopent-2-ène-1-ylhexan-2-one (composé XXII) ;
1-cyclopent-2-ène-1-ylpent-4-ène-2-one (composé XXIII) ;
(E)-1-cyclopent-2-ène-1-ylpent-3-ène-2-one (composé XXIV) ;
(Z)-1-cyclopent-2-ène-1-ylpent-3-ène-2-one (composé XXV).

6. Utilisation de mélanges selon l'une quelconque des revendications 1 à 5 comme huile parfumée.

7. Utilisation d'un mélange odorant selon au moins l'une des revendications 1 à 5 précédentes pour générer, transmettre, modifier ou renforcer une odeur fruitée de type pomme, poire, ananas, banane et fraise.

8. Procédé de préparation d'un mélange odorant, comprenant les étapes suivantes :
(a) fournir un composé de formule générale (I) 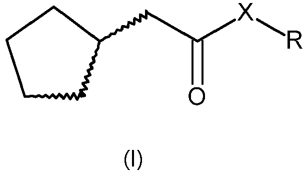 dans laquelle, pour le composé de formule (I) ou chaque composé de formule (I), X est un atome d'oxygène ou un groupe méthylène, à condition que
(i) aucune ou une des trois lignes ondulées ne représente une double liaison et les lignes ondulées restantes représentent chacune une simple liaison, et que
(ii) R représente un radical hydrocarboné linéaire ou ramifié, cyclique, saturé ou insaturé, ayant de 1 à 5 atomes de carbone, et
(b) fournir au moins un autre parfum choisi dans le groupe formé par le cantryle, le péonile, la menthone, le frescomenthe, l'isooctanone, le tolylacétaldéhyde, le phénylacétaldéhyde, le cyclogalbanate, le cinnamate d'éthyle ou leurs mélanges, et éventuellement
(c) fournir au moins un ou plusieurs parfums supplémentaires choisis dans le groupe constitué par les cétones et les esters ou les lactones ayant une masse molaire dans la plage de 220 g/mol à 320 g/mol, et
(d) mélanger des ingrédients (a), (b) et éventuellement (c).

9. Procédé pour renforcer la fraîcheur et/ou l'éclat naturel et/ou pour masquer ou atténuer les notes grasses et/ou métalliques d'un ou plusieurs composé(s) de parfum(s) autre(s) que ceux de formule générale (I), comprenant l'étape suivante :
mélanger des substances odorantes choisies dans les groupes formés par
(b) le cantryle, le péonile, la menthone, le frescomenthe, l'isooctanone, le tolylacétaldéhyde, le phénylacétaldéhyde, le cyclogalbanate, le cinnamate d'éthyle ou leurs mélanges et/ou
(c) un ou plusieurs autres parfums choisis dans le groupe constitué par les cétones et les esters ou les lactones ayant une masse molaire dans la gamme de 220 g/mol à 320 g/mol
avec une quantité de composé de formule (I) suffisante pour [renforcer la fraîcheur naturelle et/ou l'éclat du ou des parfums différents du ou des composés de formule générale (I) et/ou pour masquer ou réduire les notes grasses et/ou métalliques.

10. Procédé selon la revendication 9, dans lequel on s'établit.
(i) le rapport massique de la quantité totale de parfums b) au composé de formule (I) est supérieur ou égal à 99 :1, et/ou
(ii) le rapport massique de la quantité totale de substances odorantes c) au composé de formule (I) est supérieur ou égal à 99 :1

11. Produit parfumé contenant un mélange de substances odorantes, de préférence une huile parfumée, selon l'une des revendications 1 à 5, en une quantité sensoriellement efficace.

12. Produit parfumé selon la revendication 11, dans lequel le produit est choisi dans le groupe constitué par :
Extraits de parfum, Eaux de parfum, Eaux de toilette, Eaux de rasage, Eaux de Cologne, Produits de pré-rasage, Splash Colognes, Lingettes rafraîchissantes parfumées, Détergents acides, alcalins et neutres, Rafraîchisseurs de textiles, Aide au repassage, Détergents liquides, Détergents en poudre, Prétraitements du linge, Assouplissants du linge, Savons de lavage, Tablettes de lavage, Désinfectants, Désinfectants de surface, désodorisants, aérosols, cires et encaustiques, produits de soins personnels, crèmes et lotions pour les mains, crèmes et lotions pour les pieds, crèmes et lotions dépilatoires, crèmes et lotions après-rasage, crèmes et lotions bronzantes, produits capillaires, déodorants et antiperspirants, produits cosmétiques décoratifs, bougies, huiles pour lampes, encens, insecticides, répulsifs et carburants.

13. Produit parfumé selon les revendications 11 ou 12, **caractérisé en ce que** la proportion du mélange de substances odorantes selon l'une des revendications 1 à 5 dans le produit parfumé est de 0,01 à 10 % en poids par rapport à la masse totale du produit parfumé.
